(19) Europäisches Patentamt
European Patent Office
Office européen des brevets

(11) **EP 1 337 857 B1**

(12) **FASCICULE DE BREVET EUROPEEN**

(45) Date de publication et mention de la délivrance du brevet:
**14.01.2009 Bulletin 2009/03**

(21) Numéro de dépôt: **01998834.4**

(22) Date de dépôt: **27.11.2001**

(51) Int Cl.:
***G01N 33/68*** *(2006.01)*

(86) Numéro de dépôt international:
**PCT/FR2001/003749**

(87) Numéro de publication internationale:
**WO 2002/044735 (06.06.2002 Gazette 2002/23)**

(54) **NOUVEAU PROCEDE DE CRIBLAGE DE MODULATEURS DE LA TRANSCRIPTION BACTERIENNE**

NEUES SCREENING-VERFAHREN VON MODULATOREN DER BAKTERIELLEN TRANSKRIPTION

NOVEL METHOD FOR SCREENING BACTERIAL TRANSCRIPTION MODULATORS

(84) Etats contractants désignés:
**AT BE CH CY DE DK ES FI FR GB GR IE IT LI LU MC NL PT SE TR**

(30) Priorité: **28.11.2000 FR 0015332**

(43) Date de publication de la demande:
**27.08.2003 Bulletin 2003/35**

(73) Titulaire: **CENTRE NATIONAL DE LA RECHERCHE SCIENTIFIQUE**
**75794 Paris Cedex 16 (FR)**

(72) Inventeurs:
- **PAU, Bernard**
  **F-34080 Montpellier (FR)**
- **LEONETTI, Jean-Paul**
  **F-34170 Castelnau Le Lez (FR)**
- **ROUBY, Joëlle**
  **F-34170 Castelnau Le Lez (FR)**

(74) Mandataire: **Grosset-Fournier, Chantal Catherine**
**Grosset-Fournier & Demachy**
**54, rue Saint-Lazare**
**75009 Paris (FR)**

(56) Documents cités:
**WO-A-96/38478**

- **FERGUSON ANNA L ET AL: "Interaction of sigma70 with Escherichia coli RNA polymerase core enzyme studied by surface plasmon resonance." FEBS LETTERS, vol. 481, no. 3, 2000, pages 281-284, XP002177386 ISSN: 0014-5793**
- **MAEDA HIROTO ET AL: "Competition among seven Escherichia coli sigma subunits: Relative binding affinities to the core RNA polymerase." NUCLEIC ACIDS RESEARCH, vol. 28, no. 18, septembre 2000 (2000-09), pages 3497-3503, XP002177387 ISSN: 0305-1048**
- **STACKHOUSE T M ET AL: "RELEASE OF THE SIGMA SUBUNIT FROM ESCHERICHIA-COLI RNA POLYMERASE TRANSCRIPTION COMPLEXES IS DEPENDENT ON THE PROMOTER SEQUENCE" BIOCHEMISTRY, vol. 28, no. 19, 1989, pages 7781-7788, XP001024310 ISSN: 0006-2960**
- **SCHMIDT M C ET AL: "AMPLIFICATION AND ISOLATION OF ESCHERICHIA-COLI NUS-A PROTEIN AND STUDIES OF ITS EFFECTS ON IN-VITRO RNA CHAIN ELONGATION" BIOCHEMISTRY, vol. 23, no. 2, 1984, pages 197-203, XP001024312 ISSN: 0006-2960**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 337 857 B1

## Description

L'invention concerne un nouveau procédé de criblage de modulateurs de la transcription bactérienne, en particulier d'activateurs et d'inhibiteurs. L'invention concerne notamment un procédé de criblage d'activateurs et d'inhibiteurs de la liaison des facteurs de transcription avec l'ARN polymérase. L'invention concerne également un kit ou une trousse de détection de modulateurs de la transcription bactérienne ainsi que l'utilisation de ce procédé de criblage dans le cadre de la découverte d'antibiotiques, de médicaments antiviraux et anticancéreux.

La transcription des gènes en molécules d'ARN correspondantes est un procédé complexe catalysé par l'ARN polymérase, dépendante de l'ADN, et qui implique de nombreuses protéines.

L'ARN polymérase bactérienne se présente sous deux formes : l'enzyme minimum (ou "core enzyme") et l'holoenzyme, qui apparaît suite à la fixation du facteur de transcription sigma (σ) sur l'enzyme minimum. C'est cette holoenzyme qui reconnaît et se lie au promoteur, ce qui permet l'initiation de la transcription à partir d'un site spécifique (Burgess et al., 1969 ; Reznikoff et al., 1985). L'enzyme minimum est incapable de reconnaître les séquences du promoteur ; c'est donc l'addition d'un facteur' σ qui spécifie la localisation de l'initiation de la transcription. Cette complexation entre le facteur σ et l'ARN polymérase minimum est indispensable lors des premières étapes de la transcription bactérienne. Après ces étapes d'initiation, σ quitte l'enzyme minimum et d'autres protéines, comme NusA, se lient à l'enzyme minimum.

D'une façon générale, les facteurs σ appartiennent à une famille de protéines qui possèdent les mêmes fonctions : ce sont des sous-umités de l'ARN polymérase, nécessaires à l'initiation de la transcription ; ces facteurs ont une importance primordiale quant à la sélection des sites de liaison de l'enzyme au niveau des promoteurs.

Les facteurs NusA s'associent à l'ARN polymérase et favorisent les pauses ou la terminaison de la transcription au niveau de certaines séquences d'ADN.

Par "pauses de la transcription", on définit de façon classique un ralentissement ou un arrêt transitoire de l'activité enzymatique.

La recherche de nouvelles cibles pour des antibiotiques est une priorité pour garder une avance sur l'apparition de plus en plus fréquente de bactéries résistantes ou multirésistantes aux antibiotiques commercialisés (Courvalin, 1996).

L'ARN polymérase procaryote est une cible importante. En effet, elle est vitale pour la bactérie et elle est déjà la cible d'antibiotiques utilisés en thérapeutique (rifampicine et dérivés). Elle est également une cible pour de nombreux microorganismes (Yang et al., 1995) et bactériophages (Kolesky et al., 1999) qui luttent contre les bactéries. La recherche de nouvelles cibles sur la polymérase est donc envisageable et souhaitable.

Les ARN, les ADN polymérases ainsi que les transcriptases inverses eucaryotes, procaryotes et virales sont de bonnes cibles pour affecter le fonctionnement d'un organisme vivant. Ces activités enzymatiques sont en général relativement faciles à suivre, elles sont donc devenues des cibles de choix pour la recherche de nouvelles drogues antivirales, anticancéreuses ou antibiotiques. Cette forte activité industrielle a généré des tests d'activité plus rapides et plus fiables, adaptables aux nouvelles exigences du criblage haut débit.

Ainsi, le brevet américain n° 5 635 349 au nom de Tularik décrit une méthode d'identification d'un inhibiteur de l'activité d'une polymérase, en particulier l'ARN polymérase, dérivée par exemple d'un organisme pathogène infectieux. Cette méthode consiste à mesurer l'activité ARN polymérase en présence de molécules diverses dont on teste la capacité d'inhibition de l'activité de l'enzyme. A ce jour, la technique de base utilisée par la plupart des laboratoires consiste à mesurer l'incorporation de nucléotides radioactifs, témoins de l'activité de l'enzyme, en présence des inhibiteurs potentiels (Wu et al., 1997). Cette méthode permet de mettre en évidence globalement tous les inhibiteurs de la transcription (spécifiques comme la rifampicine, ou moins spécifiques comme les agents intercalants, les chélateurs d'ions divalents...).

Cependant, ces tests d'activité ont un coût élevé et ils sont faussés par exemple par la présence d'ARNases et d'ADNases ainsi que d'agents interagissant avec l'ADN.

Le brevet WO 96/38478 mentionne un procédé de détection de composés qui ont la capacité à inhiber l'association d'une sous-unité sigma avec l'ARN polymérase de *Mycobacterium tuberculosis*, laquelle méthode comprend la mise en contact d'un composé, avec la sous-unité sigma et l'ARN polymérase et la détection du complexe formé entre l'ARN polymérase et la sous-unité sigma. Dans ce cas, la méthode de détection a lieu par chromatographie ou par immunoprécipitation selon Lesley et al (1989) mais ces techniques de détection ne sont pas assez rapides pour faire du criblage haut débit.

A ce jour, aucun des procédés de criblage à haut débit de l'art antérieur ne permet de mettre en évidence un composé qui affecte spécifiquement une étape de la transcription pour laquelle il n'y a pas encore d'inhibiteur précédemment décrit. A ce jour, aucun des procédés de l'art antérieur ne permet la mise en évidence facile de modulateurs de la liaison entre le facteur sigma et l'ARN polymérase, lesquels ne peuvent pas être mis en évidence par transcription *in vitro*. En effet, lors de la transcription *in vitro*, le complexe entre le facteur sigma et l'ARN polymérase étant déjà formé, il n'est pas possible de déterminer des modulateurs de la liaison entre les deux molécules car cette liaison a déjà eu lieu, avant l'intervention des modulateurs potentiels.

Or, l'invention permet notamment d'apporter une solution à ces problèmes.

Le but de l'invention est de fournir un nouveau procédé rapide et industriellement applicable permettant le criblage de produits intervenant au cours de la transcription.

Le but de l'invention est de fournir un tel procédé qui soit utilisable dans le cadre d'un criblage à haut débit.

Le but de l'invention est de fournir un nouveau test de complexation dans lequel les ARNases et les ADNases n'interfèrent pas avec la mesure de l'activité transcriptionnelle, en dégradant la matrice d'ADN ou l'ARN produit.

Le but de l'invention est de fournir un nouveau procédé qui, en ciblant l'interface de complexation entre deux protéines, par exemple l'ARN polymérase et le facteur sigma, permet de limiter les risques de résistance par mutation de la cible.

L'invention concerne un procédé de détection d'un composé modulateur de la complexation entre l'ARN polymérase et une protéine intervenant au cours de la transcription, dans lequel :

- on incube un mélange comprenant l'ARN polymérase, une protéine intervenant au cours de la transcription et le composé soumis au procédé de détection, l'étape d'incubation étant effectuée dans des conditions permettant :
  - la formation d'un complexe entre l'ARN polymérase et la susdite protéine et,
  - la formation d'une liaison d'une part entre le susdit composé et d'autre part l'ARN polymérase et/ou la protéine intervenant au cours de la transcription,
- on détecte, par un test de complexation, la variation significative éventuelle de la quantité de complexe formé entre l'ARN polymérase et la susdite protéine par rapport à une valeur de contrôle correspondant à la quantité de complexe formé entre l'ARN polymérase et la susdite protéine en l'absence de tout modulateur, et
- on en déduit, lorsqu'il y a une variation significative telle que définie ci-dessus, qu'il y a eu liaison entre d'une part le susdit composé et d'autre part l'ARN polymérase et/ou la protéine intervenant au cours de la transcription, ce qui se traduit par une modulation de la complexation entre l'ARN polymérase et la protéine intervenant au cours de la transcription.

Par "protéine intervenant au cours de la transcription", on désigne tout facteur protéique qui interagit physiquement avec l'ARN polymérase ($\alpha_2$, $\beta$, $\beta'$) et modifie son activité transcriptionnelle.

On désigne par "composé modulateur de la complexation entre l'ARN polymérase et une protéine intervenant au cours de la transcription" :

- soit un composé qui provoque une diminution de la quantité de complexe formé entre l'ARN polymérase et la protéine intervenant au cours de la transcription,
- soit au contraire un composé qui provoque une augmentation de la quantité de complexe formé entre l'ARN polymérase et la protéine intervenant au cours de la transcription.

Dans ce qui suit, on désigne par partenaires les deux éléments qui constituent le complexe. On désigne par premier partenaire celui qui apparaît en premier lieu dans le mélange et par deuxième partenaire celui qui intervient chronologiquement après le premier partenaire. Dans le cas très particulier de l'addition simultanée des deux éléments qui constituent le complexe, il va de soi que les deux partenaires correspondent indifféremment au premier partenaire et au deuxième partenaire.

Par "test de complexation", on comprend une technique de révélation quantitative du complexe formé entre l'ARN polymérase et la protéine intervenant au cours de la transcription.

De façon avantageuse, ce test implique le marquage moléculaire d'au moins un des partenaires, à savoir l'ARN polymérase et/ou ladite protéine, par une substance. Ce marquage permet une mesure physique quantitative directe ou indirecte, par émission ou consommation de signal, spontanément ou après addition d'un substrat ou d'un signal.

De façon avantageuse, ce test n'implique pas la présence, pour le complexe formé entre l'ARN polymérase et ladite protéine, d'une propriété physico-chimique particulière, telle que la taille moléculaire ou le point iso-électrique. Par conséquent, ce test est différent d'une chromatographie, qu'il s'agisse d'une technique par filtration/exclusion ou par effet de charge.

Le marquage, tel que mentionné ci-dessus, peut s'effectuer en utilisant notamment un élément radioactif, un élément fluorescent, un élément luminescent, une enzyme, de la biotine pour une révélation indirecte par de l'avidine marquée, etc...

Dans un mode de réalisation particulier, lorsque les deux partenaires, à savoir l'ARN polymérase et ladite protéine, peuvent être marqués par des substances capables entre elles d'échanges énergétiques (transferts), la détermination de la quantité de complexe (ou de sa variation) peut être faite en solution ; la formation du complexe s'accompagne du rapprochement des deux partenaires, ce qui permet le transfert d'énergie entre les deux marqueurs et

entraîne alors une augmentation ou une diminution de l'intensité du signal de fluorescence émis par l'un des deux marqueurs.

Dans un autre mode de réalisation, seul l'un des partenaires est marqué et l'autre est immobilisé sur une phase solide, soit préalablement à la mise en contact avec le partenaire marqué, soit ultérieurement. L'immobilisation peut être de nature physico-chimique, comme par exemple, par adsorption sur une surface plastique hydrophobe, ou de nature bio-spécifique : dans ce cas, un attracteur biologique, qui peut être un anticorps spécifique de l'un des partenaires ou de l'avidine capable d'immobiliser le partenaire préalablement revêtu de biotine, est lui-même préalablement immobilisé. Dans tous les cas, c'est le second partenaire qui permet de déterminer la quantité de complexe ou la variation de quantité de complexe formé entre l'ARN polymérase et ladite protéine par révélation quantitative de son marqueur, qui peut avoir été fixé par liaison chimique permanente (élément radioactif, élément fluorescent, élément luminescent, enzyme, biotine...) ou être introduit de façon bio-spécifique. Dans ce cas, il est possible d'utiliser un anticorps dirigé contre le second partenaire s'il est marqué directement ou indirectement ou bien de l'avidine marquée directement ou indirectement.

Par ailleurs, ce test est également indépendant des techniques immunologiques dites ELISA (enzyme-linked immunosorbent assay) puisqu'il utilise un anticorps uniquement pour révéler l'un des partenaires d'une interaction bio-spécifique à laquelle cet anticorps est étranger. Ce test repose sur l'interaction entre l'ARN polymérase et ladite protéine, contrairement à un test ELISA, qui repose sur une interaction antigène-anticorps. De plus, dans ce test, l'anticorps est uniquement utilisé pour la détection et peut être remplacé, par exemple, par un marqueur fluorescent ou radioactif. Ce test est également différent d'une immunoprécipitation car l'anticorps n'est pas utilisé pour immunoprécipiter un complexe formé entre l'ARN polymérase et la protéine intervenant au cours de la transcription : il sert soit à capturer ce complexe sur une phase solide, soit à révéler l'un des partenaires du complexe.

Pour obtenir la valeur de contrôle, correspondant à une absence de modulation, on effectue l'expérience suivante :

- on incube un mélange comprenant l'ARN polymérase et une protéine telle que définie ci-dessus dans des conditions permettant la formation d'un complexe entre l'ARN polymérase et la susdite protéine, et
- on détecte la quantité de complexe formé entre l'ARN polymérase et la susdite protéine ; cette quantité correspondant à ladite valeur de contrôle.

Par "variation significative de la quantité de complexe formé entre l'ARN polymérase et la protéine intervenant au cours de la transcription", on désigne une variation d'environ plus de 20% de la quantité de complexe formé, et de préférence d'au moins environ 50%.

L'invention concerne un procédé de détection tel que défini ci-dessus, dans lequel le composé modulateur est un composé activateur de la complexation entre l'ARN polymérase et une protéine intervenant au cours de la transcription, et dans lequel :

- on incube un mélange comprenant l'ARN polymérase, une protéine intervenant au cours de la transcription et le composé soumis au procédé de détection, l'étape d'incubation étant effectuée dans des conditions permettant :
    - la formation d'un complexe entre l'ARN polymérase et la susdite protéine et,
    - éventuellement la formation d'une liaison d'une part entre le susdit composé et d'autre part l'ARN polymérase et/ou la protéine intervenant au cours de la transcription,
- on détecte, par un test de complexation, la variation significative éventuelle de la quantité de complexe formé entre l'ARN polymérase et la susdite protéine par rapport à une valeur de contrôle correspondant à la quantité de complexe formé entre l'ARN polymérase et la susdite protéine en l'absence de tout activateur, et
- on en déduit, lorsqu'il y a une variation significative telle que définie ci-dessus, qu'il y a eu liaison entre d'une part le susdit composé et d'autre part l'ARN polymérase et/ou la protéine intervenant au cours de la transcription, ce qui se traduit par une activation de la complexation entre l'ARN polymérase et la protéine intervenant au cours de la transcription.

Par "composé activateur de la complexation entre l'ARN polymérase et une protéine intervenant au cours de la transcription", on désigne un composé qui provoque une augmentation de la quantité de complexe.

Ledit composé activateur provoque une complexation supérieure, c'est-à-dire une augmentation d'au moins 120% et de préférence supérieure à 150% par rapport au contrôle (pourcentage de 100%) correspondant à la quantité de complexe formé entre l'ARN polymérase et la susdite protéine en l'absence de tout activateur.

L'invention concerne un procédé de détection tel que défini ci-dessus, dans lequel le composé modulateur est un composé inhibiteur de la complexation entre l'ARN polymérase et une protéine intervenant au cours de la transcription,

et dans lequel :

- on incube un mélange comprenant l'ARN polymérase, une protéine intervenant au cours de la transcription et le composé soumis au procédé de détection, l'étape d'incubation étant effectuée dans des conditions permettant :
  - la formation d'un complexe entre l'ARN polymérase et la susdite protéine et,
  - éventuellement la formation d'une liaison d'une part entre le susdit composé et d'autre part l'ARN polymérase et/ou la protéine intervenant au cours de la transcription,
- on détecte, par un test de complexation, la variation significative éventuelle de la quantité de complexe formé entre l'ARN polymérase et la susdite protéine par rapport à une première valeur de contrôle et/ou à une deuxième valeur de contrôle, l'une de ces valeurs contrôlé correspondant à la quantité de complexe formé entre l'ARN polymérase et la susdite protéine en l'absence de tout inhibiteur et l'autre de ces valeurs contrôle correspondant à la quantité de complexe formé entre l'ARN polymérase et la susdite protéine en présence d'un inhibiteur de référence, et
- on en déduit, lorsqu'il y a une variation significative telle que définie ci-dessus, qu'il y a eu formation d'une liaison entre d'une part le susdit composé et d'autre part l'ARN polymérase et/ou la protéine intervenant au cours de la transcription, ce qui se traduit par une inhibition de la complexation entre l'ARN polymérase et la protéine intervenant au cours de la transcription.

Par "composé inhibiteur de la complexation entre l'ARN polymérase et une protéine intervenant au cours de la transcription", on désigne un composé qui provoque la diminution de la quantité de complexe formé entre l'ARN polymérase et la protéine intervenant au cours de la transcription.

Pour obtenir la première valeur de contrôle, on effectue l'expérience telle que décrite ci-dessus, correspondant à une absence d'inhibition.

Pour obtenir la deuxième valeur de contrôle, correspondant à une inhibition de référence, on effectue l'expérience suivante :

- on incube un mélange comprenant l'ARN polymérase, une protéine telle que définie ci-dessus et l'inhibiteur de référence, l'étape d'incubation étant effectuée dans des conditions permettant :
  - la formation d'un complexe entre l'ARN polymérase et la susdite protéine et,
  - la formation d'une liaison entre l'inhibiteur de référence et l'ARN polymérase,
- on détecte la quantité de complexe formé entre l'ARN polymérase et la susdite protéine ; cette quantité correspondant à la deuxième valeur de contrôle. Cette quantité sera inférieure à celle mesurée lors du premier contrôle en raison de la formation du complexe entre l'inhibiteur de référence et l'ARN polymérase et de sa conséquence négative sur la formation dudit complexe.

L'inhibiteur de référence est par exemple un anticorps monoclonal, notamment l'anticorps monoclonal 3E10 (cf exemple).

Un procédé de détection avantageux est un procédé de détection tel que défini ci-dessus comportant l'utilisation d'une seule valeur de contrôle, correspondant à l'incubation d'ARN polymérase seule avec la protéine intervenant au cours de la transcription en l'absence de tout inhibiteur (ce qui correspond à une absence d'inhibition).

Pour obtenir cette valeur de contrôle, correspondant à une absence d'inhibition, on effectue l'expérience telle que décrite ci-dessus, qui comprend l'incubation d'ARN polymérase seule avec la protéine intervenant au cours de la transcription.

Un procédé de détection avantageux est un procédé tel que défini ci-dessus, comportant l'utilisation de deux valeurs contrôle, l'une correspondant à l'incubation d'ARN polymérase seule avec la protéine intervenant au cours de la transcription en l'absence de tout inhibiteur (ce qui correspond à une absence d'inhibition) et l'autre correspondant à une incubation de l'ARN polymérase avec la protéine intervenant au cours de la transcription et avec un inhibiteur de référence (ce qui correspond à une inhibition de référence).

Pour obtenir les deux valeurs contrôle, on effectue les deux expériences telles que décrites ci-dessus ; la première est obtenue en détectant la quantité de complexe formé entre l'ARN polynérase et la protéine intervenant au cours de la transcription en l'absence d'inhibiteur et la deuxième en détectant la quantité de complexe formé entre l'ARN polymérase et la protéine intervenant au cours de la transcription en présence de l'inhibiteur de référence.

Un procédé de détection avantageux est un procédé tel que défini ci-dessus, en phase solide, comportant l'utilisation d'une première valeur de contrôle et/ou d'une deuxième valeur de contrôle et/ou d'une troisième valeur de contrôle;

l'une correspondant à la quantité de complexe formé entre l'ARN polymérase et la susdite protéine en l'absence de tout inhibiteur,
l'autre correspondant à la quantité de complexe formé entre l'ARN polymérase et la susdite protéine en présence d'un inhibiteur de référence,
et l'autre correspondant :

- soit à l'absence de complexe formé entre l'ARN polymérase et la protéine intervenant au cours de la transcription, résultant de la mise en présence de la protéine intervenant au cours de la transcription fixée sur un support, de l'ARN polymérase et d'un excès de protéine intervenant au cours de la transcription, l'excès de protéine intervenant au cours de la transcription étant de préférence préalablement incubé avec l'ARN polymérase, l'absence du susdit complexe correspondant à une inhibition totale de la complexation entre l'ARN polymérase et la susdite protéine,
- soit à l'absence de complexe formé entre l'ARN polymérase et la protéine intervenant au cours de la transcription, résultant de la présence seule d'ARN polymérase et de l'absence de protéine intervenant au cours de la transcription.

Par procédé en phase solide, on désigne un procédé où l'un des partenaires, à savoir l'ARN polymérase ou la protéine intervenant au cours de la transcription, est immobilisé de façon covalente (réaction chimique) ou non covalente (adsorbtion non spécifique sur plastique, système avidine-biotine, anticorps) sur un support solide. Le second partenaire est incubé dans des conditions permettant la complexation entre les deux partenaires puis l'excès du second partenaires est éventuellement éliminé par lavage. Le complexe est ensuite soumis au procédé de détection.
Pour obtenir la première valeur de contrôle, on effectue l'expérience telle que décrite ci-dessus, correspondant à une absence d'inhibition.
Pour obtenir la deuxième valeur de contrôle, correspondant à une inhibition de référence, on détecte la quantité de complexe formé entre l'ARN polymérase et la protéine intervenant au cours de la transcription, en présence de l'inhibiteur de référence.
Pour obtenir la troisième valeur de contrôle, correspondant à une inhibition de référence,

- soit on effectue l'expérience suivante correspondant à une incubation d'un support sur lequel est fixée la protéine intervenant au cours de la transcription avec de l'ARN polymérase et un excès de la protéine intervenant au cours de la transcription, l'excès de ladite protéine étant de préférence préincubé avec l'ARN polymérase, et qui provoque une inhibition totale de la complexation entre l'ARN polymérase et la susdite protéine, c'est-à-dire une absence de complexe formé entre l'ARN polymérase et la susdite protéine,
- soit on effectue l'expérience suivante correspondant à une incubation d'un support avec l'ARN polymérase seule, ce qui entraîne une absence totale de la complexation entre l'ARN polymérase et la susdite protéine, c'est-à-dire une absence de complexe formé entre l'ARN polymérase et la susdite protéine.

Un procédé de détection avantageux est un procédé tel que défini ci-dessus, en phase liquide, comportant l'utilisation d'une première valeur de contrôle et/ou d'une deuxième valeur de contrôle et/ou d'une troisième valeur de contrôle,

l'une correspondant à la quantité de complexe formé entre l'ARN polymérase et la susdite protéine en l'absence de tout inhibiteur,
l'autre correspondant à la quantité de complexe formé entre l'ARN polymérase et la susdite protéine en présence d'un inhibiteur de référence,
et l'autre correspondant à l'absence de complexe formé entre l'ARN polymérase et la protéine intervenant au cours de la transcription, résultant de la mise en présence de l'ARN polymérase, d'un excès de protéine non marquée intervenant au cours de la transcription et de ladite protéine marquée, l'absence du susdit complexe correspondant à une inhibition totale de la complexation entre l'ARN polymérase et la susdite protéine.

Par procédé en phase liquide, on désigne un procédé où les partenaires sont en solution dans une solution tampon. L'un des partenaires ou les deux sont, par exemple, marqués avec une molécule fluorescente. Leur interaction est quantifiée par transfert ou polarisation de fluorescence.
Pour obtenir la première valeur de contrôle, on effectue l'expérience telle que décrite ci-dessus, correspondant à une absence d'inhibition.
Pour obtenir la deuxième valeur de contrôle, correspondant à une inhibition de référence, on détecte la quantité de complexe formé entre l'ARN polymérase et la protéine intervenant au cours de la transcription, en présence de l'inhibiteur de référence.

Pour obtenir la troisième valeur de contrôle, correspondant à une inhibition de référence, on effectue l'expérience suivante correspondant à une incubation d'ARN polymérase, d'un excès de protéine non marquée intervenant au cours de la transcription et de la protéine intervenant au cours de la transcription, ce qui provoque une inhibition totale de la complexation entre l'ARN polymérase et la susdite protéine, c'est-à-dire une absence de complexe formé entre l'ARN polymérase et la susdite protéine.

Un procédé de détection avantageux est un procédé tel que défini ci-dessus, dans lequel le mélange comprenant l'ARN polymérase, une protéine intervenant au cours de la transcription et un composé soumis au procédé de détection est préparé :

- soit en ajoutant simultanément l'ARN polymérase, la protéine intervenant au cours de la transcription et le composé soumis au procédé de détection,
- soit en ajoutant successivement : l'ARN polymérase, le composé soumis au procédé de détection et la protéine intervenant au cours de la transcription, ou successivement : la protéine intervenant au cours de la transcription, le composé soumis au procédé de détection et l'ARN polymérase,
- soit en ajoutant ledit composé préalablement incubé avec l'ARN polymérase ou ladite protéine, et respectivement soit ladite protéine soit l'ARN polymérase,
- soit en ajoutant ledit composé et l'ARN polymérase préalablement incubé avec ladite protéine.

La préparation du mélange comprenant l'ARN polymérase, une protéine intervenant au cours de la transcription et un composé soumis au procédé de détection en ajoutant simultanément l'ARN polymérase, la protéine intervenant au cours de la transcription et le composé soumis au procédé de détection, peut permettre de rechercher les composés qui se lient à un complexe entre l'ARN polymérase et ladite protéine et qui dissocient le susdit complexe ainsi que les composés liant seulement l'un des deux partenaires, mais suffisamment efficaces pour entrer en compétition avec un complexe préformé.

La préparation du mélange comprenant l'ARN polymérase, une protéine intervenant au cours de la transcription et un composé soumis au procédé de détection en ajoutant successivement : l'ARN polymérase, le composé soumis au procédé de détection et la protéine intervenant au cours de la transcription, peut permettre de faciliter la détection de composés qui lient l'ARN polymérase. Ce mode de réalisation peut permettre de sélectionner, outre les ligands de l'ARN polymérase, les meilleurs ligands de ladite protéine qui sont, dans ce cas, désavantagés du point de vue cinétique.

La préparation du mélange comprenant l'ARN polymérase, une protéine intervenant au cours de la transcription et un composé soumis au procédé de détection en ajoutant successivement : la protéine intervenant au cours de la transcription, le composé soumis au procédé de détection et l'ARN polymérase, peut favoriser la détection de composés qui lient la molécule intervenant au cours de la transcription ainsi que la recherche de très bons ligands de l'ARN polymérase qui sont défavorisés du point de vue cinétique.

La préparation du mélange comprenant l'ARN polymérase, une protéine intervenant au cours de la transcription et un composé soumis au procédé de détection en ajoutant ledit composé préalablement incubé avec l'ARN polymérase à ladite protéine, peut faciliter la liaison dudit composé avec l'ARN polymérase avant l'ajout de la protéine intervenant au cours de la transcription. Ce mode de réalisation comprenant une préincubation peut favoriser la détection de molécules qui lient l'ARN polymérase.

La préparation du mélange comprenant l'ARN polymérase, une protéine intervenant au cours de la transcription et un composé soumis au procédé de détection en ajoutant ledit composé préalablement incubé avec ladite protéine à l'ARN polymérase, permet de faciliter la liaison entre ledit composé et la protéine intervenant au cours de la transcription avant l'ajout de l'ARN polymérase. Ce mode de réalisation peut favoriser la détection de molécules qui lient ladite protéine.

La préparation du mélange comprenant l'ARN polymérase, une protéine intervenant au cours de la transcription et un composé soumis au procédé de détection en ajoutant ledit composé et l'ARN polymérase préalablement incubée avec ladite protéine, permet de détecter les composés qui favorisent la dissociation du complexe formé entre l'ARN polymérase et ladite protéine.

L'invention concerne également un procédé de détection tel que défini ci-dessus, dans lequel on dépose sur un support solide, avant, pendant ou après l'étape d'incubation, soit l'ARN polymérase soit la protéine intervenant au cours de la transcription.

Ce dépôt peut être opéré par intervention d'un lien covalent (physico-chimique) ou biospécifique entre le support solide et l'ARN polymérase ou ladite protéine.

Lorsqu'on dépose, avant l'étape d'incubation, l'ARN polymérase sur un support solide, il est possible que l'ARN polymérase soit dénaturée.

Lorsqu'on dépose, avant l'étape d'incubation, la protéine intervenant au cours de la transcription sur un support solide, il est possible que ladite protéine soit dénaturée.

Un procédé de détection avantageux de l'invention est un procédé tel que défini ci-dessus, dans lequel on ajoute simultanément, non préalablement mélangés, l'ARN polymérase et le composé soumis au procédé de détection,

à la protéine intervenant au cours de la transcription déposée sur un support.

Ce mode de réalisation peut permettre de détecter à la fois les ligands de la protéine intervenant au cours de la transcription, ceux de l'ARN polymérase et également ceux du complexe formé entre ladite protéine et l'ARN polymérase. Ce mode de réalisation est très stringent pour les molécules qui inhibent l'association entre l'ARN polymérase et ladite protéine et peut également permettre de rechercher des composés qui dissocient le complexe formé entre l'ARN polymérase et ladite protéine.

Un procédé de détection avantageux de l'invention est un procédé tel que défini ci-dessus, dans lequel on ajoute le composé soumis au procédé de détection préalablement incubé avec l'ARN polymérase à la protéine intervenant au cours de la transcription déposée sur un support.

Ce mode de réalisation peut permettre de détecter à la fois les ligands de la protéine intervenant au cours de la transcription, ceux de l'ARN polymérase et également ceux du complexe formé entre ladite protéine et l'ARN polymérase. Ce mode de réalisation peut faciliter la liaison dudit composé avec l'ARN polymérase mais peut également servir à sélectionner les meilleurs ligands de ladite protéine qui sont, dans ce cas, cinétiquement désavantagés.

Un procédé de détection avantageux de l'invention est un procédé tel que défini ci-dessus, dans lequel on ajoute simultanément, non préalablement mélangés, la protéine intervenant au cours de la transcription et le composé soumis au procédé de détection, à l'ARN polymérase déposée sur un support.

Ce mode de réalisation peut permettre de détecter à la fois les ligands de la protéine intervenant au cours de la transcription, ceux de l'ARN polymérase et également ceux du complexe formé entre ladite protéine et l'ARN polymérase. Ce mode de réalisation est très stringent pour les molécules qui inhibent l'association entre l'ARN polymérase et ladite protéine et peut également permettre de rechercher des composés qui dissocient le complexe formé entre l'ARN polymérase et ladite protéine.

Un procédé de détection avantageux de l'invention est un procédé tel que défini ci-dessus, dans lequel on ajoute le composé soumis au procédé de détection préalablement incubé avec la protéine intervenant au cours de la transcription à l'ARN polymérase déposée sur un support.

Ce mode de réalisation peut permettre de détecter à la fois les ligands de la protéine intervenant au cours de la transcription, ceux de l'ARN polymérase et également ceux du complexe formé entre ladite protéine et l'ARN polymérase. Ce mode de réalisation peut faciliter ainsi la liaison dudit composé avec ladite protéine avant l'incubation avec l'ARN polymérase et permet de rechercher des ligands de ladite protéine. Il peut servir à sélectionner les meilleurs ligands de l'ARN polymérase qui sont, dans ce cas, cinétiquement désavantagés.

Un procédé de détection avantageux est un procédé tel que défini ci-dessus, dans lequel on ajoute l'un après l'autre le composé soumis au procédé de détection et l'ARN polymérase à la protéine intervenant au cours de la transcription déposée sur un support.

Le dépôt de la protéine intervenant au cours de la transcription sur un support puis l'addition successive du composé soumis au procédé de détection et l'ARN polymérase, peut permettre de détecter les composés inhibant uniquement la protéine intervenant au cours de la transcription. En effet, si le composé tel que défini ci-dessus ne se fixe pas sur la protéine telle que définie ci-dessus, il est éliminé lors du lavage qui a eu lieu avant l'addition de l'ARN polymérase.

Un procédé de détection avantageux est un procédé tel que défini ci-dessus, dans lequel on ajoute l'un après l'autre le composé soumis au procédé de détection et la protéine intervenant au cours de la transcription à l'ARN polymérase déposée sur un support.

Le dépôt de l'ARN polymérase sur un support puis l'addition successive du composé soumis au procédé de détection et de la protéine intervenant au cours de la transcription, peut permettre de détecter les composés inhibant uniquement l'ARN polymérase. En effet, si le composé tel que défini ci-dessus ne se fixe pas sur l'ARN polymérase, il est éliminé lors du lavage qui a lieu avant l'addition de la protéine telle que définie ci-dessus.

L'invention concerne un procédé de détection tel que défini ci-dessus, dans lequel, au moment de l'étape d'incubation de l'ARN polymérase avec une protéine intervenant au cours de la transcription et avec un composé soumis au procédé de détection, une liaison se forme :

- soit entre ledit composé et entre l'ARN polymérase,
- soit entre ledit composé et entre ladite protéine,
- soit entre ledit composé, entre ladite protéine et entre l'ARN polymérase.

Lorsqu'une liaison se forme entre ledit composé et entre l'ARN polymérase, si le composé soumis au procédé de détection se fixe dans la région du site de complexation de ladite protéine, ledit composé empêche la protéine intervenant au cours de la transcription de se complexer à l'ARN polymérase tandis que la fixation dudit composé à côté du site de complexation de ladite protéine entraîne un changement de conformation et empêche également la complexation entre ladite protéine et l'ARN polymérase.

Lorsqu'une liaison se forme entre ledit composé et entre ladite protéine, si ledit composé se fixe dans la région

du site de complexation de l'ARN polymérase, ledit composé empêche l'ARN polymérase de se complexer à ladite protéine tandis que la fixation dudit composé à côté du site de complexation de l'ARN polymérase entraîne un changement de conformation et empêche également la complexation entre ladite protéine et l'ARN polymérase.

Lorsqu'une liaison se forme entre ledit composé, entre ladite protéine et entre l'ARN polymérase, soit ledit composé lie l'ARN polymérase engagée dans le complexe avec ladite protéine et favorise la dissociation, soit le composé lie ladite protéine, change sa conformation et force la dissociation.

L'invention concerne un procédé de détection tel que défini ci-dessus, dans lequel, au cours de l'étape de détection de la variation significative éventuelle de la quantité de complexe formé entre l'ARN polymérase et la protéine intervenant au cours de la transcription, on utilise un anticorps anti-ARN polymérase.

L'invention concerne un procédé de détection tel que défini ci-dessus, dans lequel la protéine intervenant au cours de la transcription a un poids moléculaire supérieur à environ 15 kDa ou est une protéine de fusion entre une protéine de poids moléculaire inférieur à 15 kDa et une autre protéine, telle que la GST (glutathione S transférase).

Des exemples de telles protéines comprennent notamment des domaines du facteur sigma sous forme de fusion GST, et les protéines Gp33 ou 55 du bactériophage T4 sous forme de fusion GST.

L'invention concerne un procédé de détection tel que défini ci-dessus, dans lequel les concentrations de l'ARN polymérase sont comprises d'environ 1 fmole à environ 100 pmole/essai, notamment d'environ 1 fmole à environ 10 pmole/essai, celles de la protéine intervenant au cours de la transcription d'environ 10 fmole à environ 500 pmole/essai et celles du composé soumis au procédé de détection d'environ 1 pM à environ 1 $\mu$M, notamment d'environ 1 nM à environ 1 $\mu$M.

Les gammes de concentrations inférieures à la concentration la plus faible correspondent à la limite de détection tandis que les gammes de concentrations supérieures à la concentration la plus forte favorisent une liaison non spécifique et demandent trop de quantité de protéine, ce qui est très défavorable concernant les concentrations de composé soumis au procédé de détection, qu'il est nécessaire d'ajouter pour observer l'effet inhibiteur.

L'invention concerne un procédé de détection tel que défini ci-dessus, dans lequel l'ARN polymérase utilisée provient de cellules procaryotes, notamment d'*E.coli*.

L'invention concerne un procédé de détection tel que défini ci-dessus, dans lequel la protéine intervenant au cours de la transcription intervient soit au cours de l'étape d'initiation de la transcription, soit au cours de l'étape d'élongation, soit au cours de l'étape de terminaison de la transcription.

Par "intervenant au cours de l'étape d'initiation de la transcription", on entend une protéine qui se lie à l'ARN polymérase, lui confère une spécificité pour un promoteur et permet l'initiation de la transcription.

Des exemples de protéines intervenant au cours de l'initiation sont notamment la famille des facteurs sigma, notamment le facteur sigma 70, ainsi que les protéines Gp33, Gp45 et Gp55 du bactériophage T4.

Par "intervenant au cours de l'étape d'élongation de la transcription", on entend une protéine qui se lie à l'ARN polymérase et change sa vitesse d'élongation.

Des exemples de protéines intervenant au cours de l'élongation sont notamment les protéines NusA, greA et greB.

Par "intervenant au cours de l'étape de terminaison de la transcription", on entend une protéine qui change le site de terminaison de la transcription.

Des exemples de protéines intervenant au cours de la terminaison sont notamment les protéines NusA, NusB, NusG, Rho ou la protéine N du bactériophage lambda.

L'invention concerne un procédé de détection tel que défini ci-dessus, dans lequel la protéine intervenant au cours de la transcription est :

- soit la protéine $\sigma^{70}$ ou une protéine équivalente,
  Par protéine équivalente, on comprend toute protéine qui lie l'ARN polymérase, lui confère une spécificité pour un promoteur et permet l'initiation.
- soit la protéine NusA ou une protéine équivalente,
  Par protéine équivalente, on comprend toute protéine ayant entre 22 et 100% d'identité de séquence avec celle de la protéine NusA d'*E.coli* (Swiss Prot P03003).
- soit des fragments d'une de ces deux protéines,
- soit une de ces deux protéines sous forme de protéine de fusion,
- soit des fragments d'une de ces deux protéines sous forme de protéine de fusion.

L'invention concerne un procédé de détection tel que défini ci-dessus, dans lequel :

- on adsorbe la protéine intervenant au cours de la transcription sur un support,
- on incube ledit support avec l'ARN polymérase et avec le composé soumis au procédé de détection, ce qui conduit à la formation d'un complexe entre l'ARN polymérase et ladite protéine et la formation éventuelle d'une liaison entre

l'ARN polymérase et le susdit composé,

- on incube ledit support avec un anticorps anti-ARN polymérase,
- on détecte, par un test de complexation, la variation significative éventuelle de la quantité de complexe formé entre l'ARN polymérase et la susdite protéine par rapport à une valeur de contrôle correspondant à la quantité de complexe formé entre l'ARN polymérase et la susdite protéine en l'absence de tout modulateur, et
- on en déduit, lorsqu'il y a une variation significative telle que définie ci-dessus, qu'il y a eu formation d'une liaison entre le susdit composé et l'ARN polymérase, ce qui correspond à une modulation de la complexation entre l'ARN polymérase et la protéine intervenant au cours de la transcription.

Par "modulation de la complexation entre l'ARN polymérase et la protéine intervenant au cours de la transcription", on comprend à la fois l'activation et l'inhibition de ladite complexation.

L'invention concerne également un procédé de détection tel que défini ci-dessus, dans lequel :

- on adsorbe la protéine intervenant au cours de la transcription sur un support,
- on incube ledit support avec l'ARN polymérase et avec le composé soumis au procédé de détection, ce qui conduit à la formation d'un complexe entre l'ARN polymérase et ladite protéine et la formation éventuelle d'une liaison entre l'ARN polymérase et le susdit composé,
- on incube ledit support avec un anticorps anti-ARN polymérase,
- on détecte, par un test de complexation, la variation significative éventuelle de la quantité de complexe formé entre l'ARN polymérase et la susdite protéine par rapport à une valeur de contrôle correspondant à la quantité de complexe formé entre l'ARN polymérase et la susdite protéine en l'absence de tout inhibiteur, et
- on en déduit, lorsqu'il y a une variation significative telle que définie ci-dessus, qu'il y a eu formation d'une liaison entre le susdit, composé et l'ARN polymérase, ce qui correspond à une inhibition de la complexation entre l'ARN polymérase et la protéine intervenant au cours de la transcription.

## FIGURES

La Figure 1 correspond à l'inhibition de la liaison entre l'enzyme minimum de l'ARN polymérase et la protéine $\sigma^{70}$ par les composés de la famille de 5 988 031, les formules chimiques desdits composés étant représentées plus loin. Ainsi, les différents composés testés sont incubés en présence d'enzyme minimum dans une boîte de microdosage sur laquelle est adsorbée la protéine $\sigma^{70}$, chaque puits de la plaque contenant 1 $\mu$M de $\sigma^{70}$ pendant la phase d'adsorption. La boîte est ensuite lavée et incubée avec l'anticorps monoclonal 11D11 marqué à la peroxydase, puis révélée.

L'axe des ordonnées représente la densité optique mesurée à 496 nm, résultant de l'inhibition du mélange comprenant l'enzyme minimum de l'ARN polymérase, la protéine $\sigma^{70}$ et un composé testé, et l'axe des abscisses représente la concentration desdits composés testés en $\mu$g/ml.

La courbe avec les étoiles (*) correspond à la densité optique à 496 nm avec le composé 5 988 031 ; la courbe avec les ronds (•) correspond à la densité optique à 496 nm avec le composé 5 951 261 ; la courbe avec les triangles (▲) correspond à la densité optique à 496 nm avec le composé 5 128 773 ; la courbe avec les carrés (■) correspond à la densité optique à 496 nm avec le composé 5 128 772 ; la courbe avec les losanges (♦) correspond à la densité optique à 496 nm avec le composé 5 128 767 et la courbe avec les croix (x) correspond à la densité optique à 496 nm avec le composé 5 210 476.

On rappelle que le pourcentage de liaison se calcule de la manière suivante :

$$\text{\% de liaison} = \frac{(\text{absorbance en présence de l'inhibiteur testé} - \text{absorbance minimale avec un excès d'inhibiteur de référence})}{(\text{absorbance maximale sans inhibiteur testé} - \text{absorbance minimale avec un excès d'inhibiteur de référence})} \times 100$$

La Figure 2 représente le pourcentage de liaison entre l'ARN polymérase et la protéine $\sigma^{70}$, par rapport à une valeur contrôle correspondant à la liaison maximale (pourcentage de liaison de 100%), pour les composés 5 858 445, 5 761 990 et 5 768 818 (voir formules plus loin).

La Figure 3 représente la densité optique mesurée à 490 nm lors de la mise en présence d'ARN polymérase et de la protéine NusA fixée sur un support avec l'un des composés testés pour sa capacité à inhiber la liaison entre l'ARN polymérase et NusA.

Plus précisément, les différents composés testés (de la famille de 5 988 031) sont incubés en présence d'enzyme minimum dans une boîte de microdosage sur laquelle est adsorbée la protéine NusA à une concentration égale à 20 $\mu$g/ml. La boîte est ensuite lavée et incubée avec l'anticorps monoclonal 11D11 marqué à la peroxydase, puis révélée.

La Figure 4 correspond à l'inhibition de la croissance de cellules *E. coli* TG1 par le composé susmentionné 5

988 031. Cette figure représente la densité optique mesurée à 650 nm (en ordonnées) en fonction de la concentration du composé 5 988 031 en μg/ml (en abscisses).

Les cellules *E. coli* TG1, diluées selon le protocole décrit ci-après dans la partie expérimentale, sont incubées avec des concentrations croissantes du composé 5 988 031. La croissance des bactéries est mesurée à 650 nm après incubation pendant 12 heures à 37°C sous agitation dans une boîte de microdosage.

## PARTIE EXPERIMENTALE

L'invention a pour objet une nouvelle méthode qui permet le criblage d'une zone d'interaction de deux protéines indispensables à la vie de la cellule afin de limiter les résistances par mutation de la cible.

L'invention a pour objet le criblage de banques de produits chimiques de synthèse ou de produits naturels, même contaminés par des activités RNases ou DNases.

## MATERIEL ET METHODES

### Détection de la liaison sigma70-polymérase

Les protéines sigma70 et l'ARN polymérase d'*E.coli* sont exprimées et purifiées dans des conditions standards (Burgess et al., Biochemistry, 1975, Oct 21 ; 14(21) : 4634-8 ; Burgess R., Methods Enzymol 1996 ; 273 : 145-9). La protéine sigma70 est stockée à -80°C dans (20 mM tris HCl pH 8 ; 5 M chlorure de guanidine ; 10 mM β-mercaptoéthanol ; 50% de glycérol à une concentration de 100 μM). L'ARN polymérase minimum est stockée à -80°C dans (20 mM tris HCl pH 8, 100 mM KCl, 10 mM β-mercaptoéthanol, 50% glycérol) à une concentration de 1 μM. L'anticorps monoclonal antipolymérase a été obtenu et purifié par des techniques classiques (Short Protocols in Molecular Biology 2nd edition, John Wiley & Son) puis a été couplé chimiquement à la peroxydase activée (Boehringer Mannheim Biochemica réf. 1428861) selon les recommandations du fournisseur.

Le test est basé sur l'adsorption de sigma70 sur une plaque d'ELISA pendant 12 heures à 4°C (6 pmoles de protéine diluée dans 100 μl de PBS par puits, sur des plaques Nunc-immuno plate, Maxisorp.). Les dilutions de protéine et les tampons ont été optimisés pour réduire la liaison non spécifique de l'ARN polymérase. Après lavage de la plaque avec du PBS 0,1% tween20, la plaque est saturée avec 200 μl de PBS 0,1% tween20, 1% BSA puis est incubée pendant 1 heure à température ambiante avec de l'ARN polymérase d'*E.coli* (0,25 pmole d'ARN polymérase minimum dans 100 μl de PBS 0,1% tween 20, 1% BSA, 10 mM $MgCl_2$) en présence ou en l'absence d'un inhibiteur éventuel. La plaque est ensuite lavée puis est incubée avec un anticorps anti-ARN polymérase couplé à la peroxydase pendant 30 minutes à température ambiante. Le complexe sigma70-ARN polymérase anticorps est détecté avec un substrat de la peroxydase, l'O-phénylènediamine, ou un autre moyen approprié.

Alternativement, un autre protocole a été utilisé. Ce test est basé sur l'adsorption d'ARN polymérase minimum sur une plaque d'ELISA pendant 12 heures à 4°C (0,5 pmoles de protéine diluée dans 100 μl de PBS par puits sur des plaques Nunc-immuno plate Maxisorp). Après lavage de la plaque avec du PBS 0,1% tween20, la plaque est saturée avec 200 μl de PBS 0,1% tween20, 1% BSA, puis est incubée pendant 1 heure à température ambiante avec sigma70 comportant une étiquette polyhistidine C-terminale (1 pmole de sigma70 dans 100 μl de PBS 0,1% tween20, 1% BSA, 10 mM $MgCl_2$) en présence ou en l'absence d'un inhibiteur éventuel. La plaque est ensuite lavée puis est incubée avec un anticorps étiquette polyhistidine couplé à la peroxydase (Sigma réf. A7058 dilué au 1/2000 (v/v)) pendant 30 minutes à température ambiante. Le complexe sigma70-ARN polymérase anticorps est détecté avec un substrat de la peroxydase, l'O-phénylènediamine ou un autre moyen approprié.

### Détection de la liaison NusA-polymérase

Le gène codant pour la protéine NusA et comportant une étiquette polyhistidine C-terminale a été cloné dans un vecteur de type pet21. Après transfection dans des cellules BL211amdaDE3, les cellules sont cultivées dans du milieu LB à 37°C sous agitation forte. La production de protéine est induite par l'addition de 1 mM IPTG. Après 3 heures de culture, les cellules sont récupérées par centrifugation et sont lysées selon (Burgess et al., Biochemistry, 1975 Oct 21 ; 14(21): 4634-8). Après centrifugation, le surnageant est passé sur une colonne de Ni NTA agarose (Quiagen) selon les recommandations du fournisseur. La protéine NusA est stockée à -80°C dans (20 mM tris HCl pH 8 ; 5 M chlorure de guanidine ; 10 mM β-mercaptoéthanol ; 50% glycérol) à une concentration de 100 μM.

Le test est basé sur l'adsorption de NusA sur une plaque d'ELISA pendant 12 heures à 4°C (6 pmoles de protéine diluée dans 100 μl de PBS par puits sur des plaques Nunc-immuno plate, Maxisorp.). Les dilutions de protéine et les tampons ont été optimisés pour réduire la liaison non spécifique de l'ARN polymérase. Après lavage de la plaque avec du PBS 0,1% tween20, la plaque est saturée avec 200μl de PBS 0,1% tween20, 1% BSA, puis est incubée pendant 1 heure à température ambiante avec de l'ARN polymérase d'*E.coli* (0,25 pmole d'ARN polymérase minimum dans 100

µl de PBS 0,1% tween20, 1% BSA, 10 mM $MgCl_2$) en présence ou en l'absence d'un inhibiteur éventuel. La plaque est ensuite lavée puis est incubée avec un anticorps anti-ARN polymérase couplé à la peroxydase pendant 30 minutes à température ambiante. Le complexe sigma70-ARN polymérase-anticorps est détecté avec un substrat de la peroxydase, l'O-phénylènediamine, ou un autre moyen approprié.

## PREPARATION DE L'INHIBITEUR DE REFERENCE ET DE L'ANTICORPS UTILISE POUR LA DETECTION

Les souris sont immunisées avec de l'ARN polymérase d'*E.coli*. Après 3 rappels avec 100 µg, 50 µg et 10 µg d'ARN polymérase en présence d'adjuvant complet de Freund, les lymphocytes de la rate de souris immunisées sont fusionnés avec les cellules du lymphome. Un groupe de 9 anticorps monoclonaux est sélectionné par ELISA en utilisant de l'ARN polymérase déposée sur des plaques. Parmi ces 9 anticorps, on obtient l'anticorps 3E10 qui est un inhibiteur de la liaison entre l'ARN polymérase et la protéine σ70 ou NusA, ainsi que l'anticorps 11D11, qui reconnaît la sous-unité β' de l'ARN polymérase, et qui peut servir de révélateur de la liaison entre l'ARN polymérase et la protéine σ70 ou NusA.

La production d'anticorps monoclonaux à partir de fluide ascite est effectuée selon les protocoles standards (*Short Protocols in Molecular Biology*). Les anticorps sont purifiés par chromatographie d'affinités sur une colonne de protéine A-sépharose selon la référence "*Short Protocols in Molecular Biology*".

## CRIBLAGE DE PRODUITS

### Criblage primaire

Un criblage en compétition entre $\sigma^{70}$, l'enzyme minimum ("core enzyme") de l'ARN polymérase d'*E. coli* et les composés chimiques d'une banque de 3200 molécules (Chembridge Inc.) a été effectué. La protéine $\sigma^{70}$, à une concentration de 1 µM dans du tampon PBS (NaCl 150 mM ; $K_2PO_4$ 2,5 mM ; $Na_2PO_4$ 8,5 mM - pH 7,2), est adsorbée sur une plaque de microdosage durant une nuit à 4°C. Les plaques sont lavées trois fois avec du PBS-T 0,1% (v/v) (NaCl 150 mM ; $K_2PO_4$ 2,5 mM ; $Na_2PO_4$ 8,5 mM - pH 7,2 ; Tween 20 0,1% (v/v)) pour éliminer tout ce qui ne s'est pas fixé sur la plaque. Pour éviter toute réaction non spécifique, les puits de la plaque sont ensuite saturés avec 200 µl de solution de saturation (PBS-T 0,1% (v/v) ; BSA 1% (p/v)) pendant une heure à température ambiante. Après élimination de la solution de saturation, l'ARN polymérase et les compétiteurs éventuels (à une concentration de 20 µg/ml) sont incubés dans ces mêmes puits pendant une heure à température ambiante. Les plaques sont ensuite lavées trois fois avec du PBS-T 0,1% (v/v). La liaison entre l'enzyme minimum et la protéine $\sigma^{70}$ est révélée par un anticorps monoclonal dirigé contre la sous-unité β de l'ARN polymérase et couplé à la peroxydase dilué au 1/2000$^{e}$ (11D11) dans le tampon de saturation et incubé pendant 30 minutes à température ambiante.

Après trois lavages avec du PBS-T 0,1% (v/v), le substrat de la peroxydase est ajouté (OPD, Biorad). La réaction se déroule pendant 2 à 3 minutes dans l'obscurité puis une mesure de l'absorbance est effectuée à 490 nm, après arrêt de la réaction avec 50 µl de $H_2SO_4$ 4N.

Pour le criblage d'inhibiteurs de l'interaction entre l'enzyme minimum de l'ARN polymérase et la protéine NusA, le protocole est identique. Cependant, l'étape d'incubation avec les inhibiteurs potentiels et l'ARN polymérase se fait dans un mélange NaCl 350 mM - $K_2PO_4$ 2,5 mM - $Na_2PO_4$ 8,5 mM ; pH 7,2, afin de limiter les liaisons non spécifiques entre l'ARN polymérase et la protéine NusA.

Les composés retenus sont ceux inhibant à plus de 50% la liaison entre l'ARN polymérase et la protéine NusA ou $\sigma^{70}$, en utilisant pour références :

- une mesure sans inhibiteur, c'est-à-dire en présence seulement de l'ARN polymérase et de la protéine intervenant au cours de la transcription, correspondant à la liaison maximale,
- une mesure en présence d'ARN polymérase et de 100 pmole de $\sigma^{70}$ ou de NusA libre, correspondant à la liaison minimale.

Ainsi, on a retenu principalement les composés suivants :

**5 128 779**

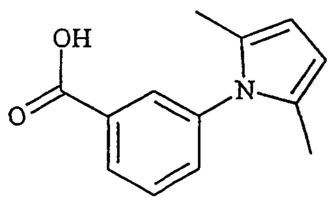

**5 128 772**

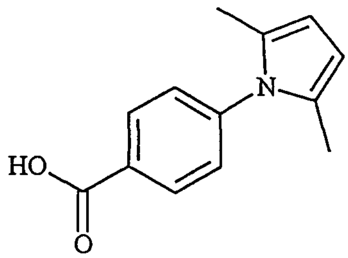

**5 210 476**

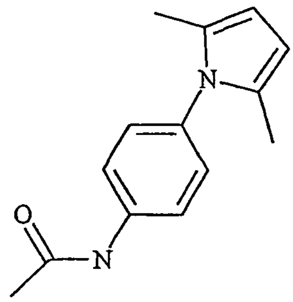

**5 988 031**

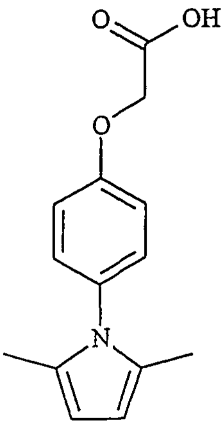

**5 128 767**

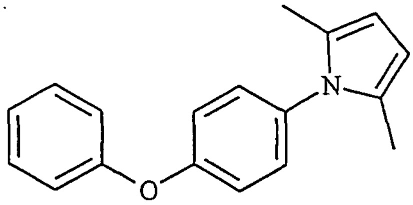

**5 951 261**

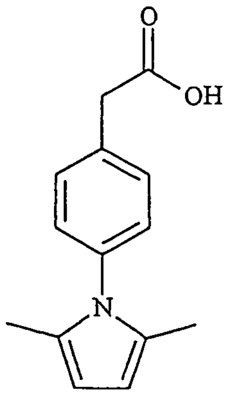

ainsi que les composés de formules :

5 858 445

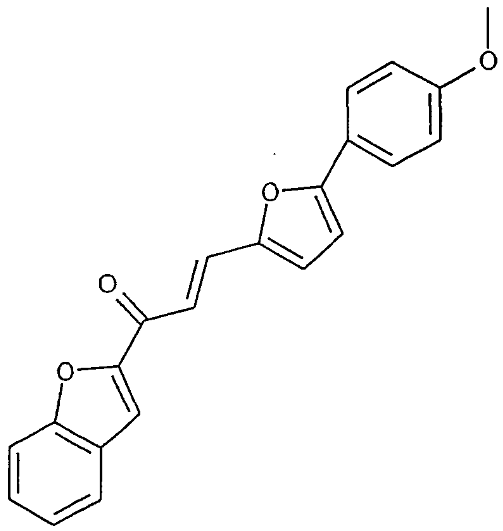

5 761 890

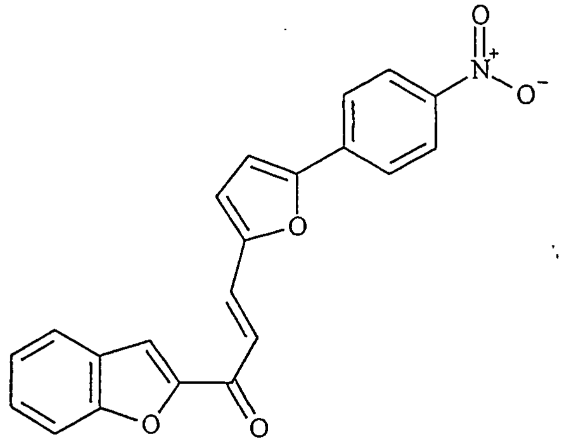

5 768 818

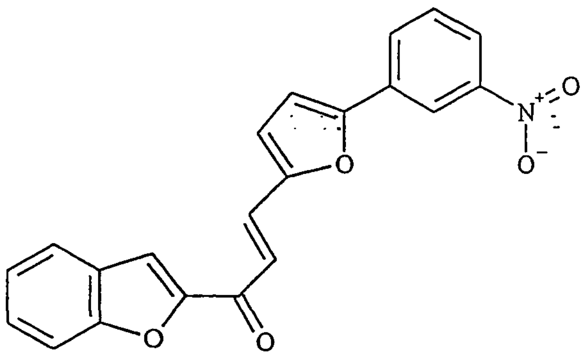

Les composés isolés à l'issue du criblage primaire sont par la suite testés à différentes concentrations (250 ; 165 ; 60 ; 30 ; 15 ; 7,5 et 3 μg/ml), selon les protocoles décrits précédemment.

**Activité auti-bactérienne**

a) Inhibition de la croissance en boîte de microdosage :

Les divers composés isolés sont testés pour leurs propriétés à inhiber la croissance de bactéries *E. coli* TG1. Des cellules en phase stationnaire sont diluées au 1/10 000 dans du milieu LB (tryptone 10 g ; extrait de levure 5 g ; NaCl 5 g) et sont transfectées dans une boîte de microdosage (200 μl/puits). Les molécules sont ajoutées dans du DMSO 10%, à la concentration finale de 100 mg/ml. Dans ces conditions, le solvant n'affecte pas la croissance des bactéries. Après une incubation d'une nuit à 37°C sous agitation, la densité optique de chaque puits à 650 nm est mesurée.

b) Inhibition de la croissance en milieu solide :

Une préculture des souches E. coli K12, S. aureus et S. epidermis en milieu Mueller Hinton Broth (Mueller and Hinton, 1941) est effectuée à 37°C, jusqu'à obtention d'une densité optique de 0,1 à 650 nm. On ajoute 100 μl de la préculture à 10 ml du milieu : agarose 0,1 %, phosphate de sodium 10 mM - pH 7,4 ; trypcase soja 0,3 mg/ml ; NaCl 100 mM.

L'ensemble est coulé sur une boîte de Pétri de 10 mm. Des puits sont effectués dans la gélose et 5 μl des solutions à tester, contenant les produits criblés, sont déposées dans chaque puits. Les boîtes sont laissées à température ambiante pendant 2 heures, puis 10 ml du milieu (agarose 1% ; phosphate de sodium 10 mM - pH 7,4 - trypcase soja 6%) sont coulés sur la boîte de Pétri formant une surcouche. Après solidification, les boîtes sont incubées pendant une nuit à 37°C.

L'activité antibactérienne est évaluée en mesurant le diamètre d'inhibition de la croissance bactérienne de la zone au centre de laquelle le produit a été déposé.

## CONCLUSION

Les résultats des tests de liaison démontrent clairement qu'il est possible de mettre en évidence des composés de petit poids moléculaire qui inhibent à des degrés divers la liaison entre l'ARN polymérase et la protéine NusA ainsi que la liaison entre l'ARN polymérase et la protéine $\sigma^{70}$ (voir Figures 1, 2 et 3).

On constate donc que les deux composés les plus actifs dans ce test de liaison sont les composés 5 988 031 (Figure 1 et 3) et 5 858 445 (Figure 2).Dans le cas du composé 5 988 031 (Figure 1), on observe une inhibition de 50% de la liaison entre $\sigma^{70}$ et l'ARN polymérase pour une concentration de $\sigma^{70}$ d'environ 5 μg/ml. On constate également que trois analogues dudit composé, à savoir les composés 5 951 261, 5 128 773 et 5 128 772, sont aussi efficaces à des concentrations 5 à 10 fois supérieures. Ainsi, la position et la longueur de la chaîne qui porte l'acide carboxylique lié au cycle benzénique du produit 5 988 031 influent fortement sur l'affinité apparente de ces molécules.

Les deux familles de molécules testées, à savoir celles du composé 5 988 031 et 5 858 445, déplacent à la fois la liaison enzyme minimum - $\sigma^{70}$ et la liaison enzyme minimum - NusA. La liaison de ces deux protéines avec l'ARN polymérase étant mutuellement exclusive (ce qui signifie que les deux protéines sont en compétition pour un même site de liaison), il n'est pas surprenant que les deux inhibiteurs testés déplacent les deux protéines. Cependant, on ne peut pas exclure qu'il soit possible d'isoler des inhibiteurs spécifiques de la liaison entre l'ARN polymérase et l'une ou l'autre de ces protéines.

Le composé 5 988 031 inhibe la liaison entre $\sigma^{70}$ et l'ARN polymérase, la liaison entre NusA et l'ARN polymérase, mais pas la liaison entre un anticorps, par exemple 11D11, et l'ARN polymérase ou l'assemblage des sous-unités $\alpha$, $\beta$ et $\beta$'. Ces résultats permettent donc de démontrer la spécificité de ce composé.

Par ailleurs, les activités antibiotiques de ces différentes molécules ont été évaluées sur des bactéries cibles.

Ainsi, le composé 5 988 031 inhibe la croissance de cellules relativement sensibles comme E. coli TG1, ce qui démontre le lien direct entre le test de liaison et l'activité biologique (voir Figure 4). Cependant, on a constaté que cette molécule n'est pas active sur d'autres souches testées telles que S. aureus, E. coli K12, M. Luteus...

Le tableau, représenté ci-dessus, correspond aux résultats obtenus lors des tests d'activité bactérienne et indique pour chaque composé testé le diamètre d'inhibition mesuré.

| | K12 | S epidermidis | S aureus |
|---|---|---|---|
| **5 858 445** 500 μg/ml | négatif | 4,2 mm | 4,2 mm |
| **5 761 890** 500 μg/ml | négatif | négatif | négatif |
| **5 768 818** 500 μg/ml | négatif | négatif | négatif |
| **vancomycine** 50 μg/ml | négatif | 5 mm | 5 mm |

Le composé 5 858 445 inhibe également fortement la croissance de cellules de bactéries S. aureus et S. Epidermidis dans le test en milieu solide (voir activité anti-bactérienne). Un diamètre d'inhibition (cf. activité anti-bactérienne) de 4,2 mm est observé à une concentration de 500 μg/ml, alors que dans ces conditions, on observe un diamètre d'inhibition de 5 mm avec de la vancomycine à 50 μg/ml.

On constate par ailleurs que les analogues de ce composé 5 858 445 n'ont aucun effet concernant l'activité anti-bactérienne. Il semble donc que le remplacement du groupement nitro par un groupement méthoxy soit important pour les propriétés anti-bactériennes des composés mais pas pour les propriétés d'inhibition de la liaison entre l'ARN polymérase et une protéine intervenant au cours de la transcription.

Ces résultats permettent de démontrer le lien entre l'activité sur le test de liaison et l'activité biologique. De plus, des résultats obtenus sur le test de liaison avec des protéines de E. coli permettent de cibler des molécules actives sur d'autres bactéries pathogènes qui ont souvent une ARN polymérase présentant de fortes homologies avec celle de E. coli, c'est-à-dire un pourcentage d'identité d'environ 90% au niveau des régions de σ impliquées dans la liaison,avec l'ARN polymérase.

## REFERENCES

- Burgess et al. (1969) Nature 221, 43-44,
- Burgess et al. (1975) Biochemistry, Oct 21 ; 14(21) : 4634-8,
- Burgess (1996) Methods Enzymol 273 : 145-9,
- Courvalin (1996) Antimicrob Chemother 37, 855-69,
- Kolesky et al. (1999) J Mol Biol 291, 267-81,
- Lesley et al. (1989) Biochemistry 28, 7728-7734,
- Mueller, J.H. and Hinton, J. (1941) A protein-free medium for primary isolation of gonococcus and meningococcus, Proc. Soc. Exp. Biol. Med. 48, 330-333,
- Reznikoff et al. (1985) Annu. Rev. Genet. 19, 355-387,
- Wu et al. (1997) Analytical Biochemistry 245, 226-230,
- Yang et al. (1995) J Biol Chem 270, 23930-3,
- Short Protocols in Molecular Biology, 2nd Edition, John Wiley & Son.


## Revendications

1. Procédé de détection d'un composé modulateur de la complexation entre l'ARN polymérase et une protéine intervenant au cours de la transcription, dans lequel :

   - on dépose sur un support solide la protéine intervenant au cours de la transcription, afin d'adsorber ladite protéine sur ledit support solide,
   - on ajoute audit support solide l'ARN polymérase et le composé soumis au procédé de détection,
   - on incube ledit support solide comprenant l'ARN polymérase, la protéine intervenant au cours de la transcription et le composé soumis au procédé de détection, et on forme un complexe entre l'ARN polymérase et la susdite protéine et une liaison d'une part entre le susdit composé et d'autre part l'ARN polymérase et/ou la protéine intervenant au cours de la transcription,
   - on détecte, par un test de complexation autre qu'une chromatographie ou qu'une immunoprécipitation, la variation significative de la quantité de complexe formé entre l'ARN polymérase et la susdite protéine par rapport à une valeur de contrôle correspondant à la quantité de complexe formé entre l'ARN polymérase et la susdite protéine en l'absence de tout modulateur, ladite variation significative désignant une variation d'environ plus de 20% de la quantité de complexe formé, et
   - on en déduit, lorsqu'il y a une variation significative telle que définie ci-dessus, qu'il y a eu liaison entre d'une part le susdit composé et d'autre part l'ARN polymérase et/ou la protéine intervenant au cours de la transcription, ce qui se traduit par une modulation de la complexation entre l'ARN polymérase et la protéine intervenant au cours de la transcription.

2. Procédé de détection selon la revendication 1, dans lequel le composé modulateur est un composé activateur de la complexation entre l'ARN polymérase et une protéine intervenant au cours de la transcription, et dans lequel :

   - on dépose sur un support solide la protéine intervenant au cours de la transcription, afin d'adsorber ladite protéine sur ledit support solide,
   - on ajoute audit support solide l'ARN polymérase et le composé soumis au procédé de détection,
   - on incube ledit support solide comprenant l'ARN polymérase, la protéine intervenant au cours de la transcription et le composé soumis au procédé de détection, et on forme un complexe entre l'ARN polymérase et la susdite

protéine et, une liaison d'une part entre le susdit composé et d'autre part l'ARN polymérase et/ou la protéine intervenant au cours de la transcription,
- on détecte, par un test de complexation autre qu'une chromatographie ou qu'une immunoprécipitation, la variation significative de la quantité de complexe formé entre l'ARN polymérase et la susdite protéine par rapport à une valeur de contrôle correspondant à la quantité de complexe formé entre l'ARN polymérase et la susdite protéine en l'absence de tout activateur, ladite variation significative désignant une augmentation d'au moins 120% de la quantité de complexe formé, et
- on en déduit, lorsqu'il y a une variation significative telle que définie ci-dessus, qu'il y a eu liaison entre d'une part le susdit composé et d'autre part l'ARN polymérase et/ou la protéine intervenant au cours de la transcription, ce qui se traduit par une activation de la complexation entre l'ARN polymérase et la protéine intervenant au cours de la transcription.

**3.** Procédé de détection selon la revendication 1, dans lequel le composé modulateur est un composé inhibiteur de la complexation entre l'ARN polymérase et une protéine intervenant au cours de la transcription, et dans lequel :

- on dépose sur un support solide la protéine intervenant au cours de la transcription, afin d'adsorber ladite protéine sur ledit support solide,
- on ajoute audit support solide l'ARN polymérase et le composé soumis au procédé de détection,
- on incube ledit support solide comprenant l'ARN polymérase, la protéine intervenant au cours de la transcription et le composé soumis au procédé de détection, et on forme un complexe entre l'ARN polymérase et la susdite protéine et, une liaison d'une part entre le susdit composé et d'autre part l'ARN polymérase et/ou la protéine intervenant au cours de la transcription,
- on détecte, par un test de complexation autre qu'une chromatographie ou qu'une immunoprécipitation, la variation significative de la quantité de complexe formé entre l'ARN polymérase et la susdite protéine par rapport à une valeur de contrôle correspondant à la quantité de complexe formé entre l'ARN polymérase et la susdite protéine en l'absence de tout inhibiteur, ladite variation significative désignant une diminution d'environ plus de 20% de la quantité de complexe formé, et
- on en déduit, lorsqu'il y a une variation significative telle que définie ci-dessus, qu'il y a eu liaison entre d'une part le susdit composé et d'autre part l'ARN polymérase et/ou la protéine intervenant au cours de la transcription, ce qui se traduit par une inhibition de la complexation entre l'ARN polymérase et la protéine intervenant au cours de la transcription.

**4.** Procédé de détection selon la revendication 3, comportant l'utilisation de deux valeurs contrôle, l'une correspondant à l'incubation d'ARN polymérase seule avec la protéine intervenant au cours de la transcription en l'absence de tout inhibiteur (ce qui correspond à une absence d'inhibition) et l'autre correspondant à une incubation de l'ARN polymérase avec la protéine intervenant au cours de la transcription et avec un inhibiteur de référence (ce qui correspond à une inhibition de référence).

**5.** Procédé de détection selon la revendication 3, comportant l'utilisation d'une première valeur de contrôle et/ou d'une deuxième valeur de contrôle et/ou d'une troisième valeur de contrôle,

l'une correspondant à la quantité de complexe formé entre l'ARN polymérase et la susdite protéine en l'absence de tout inhibiteur,
l'autre correspondant à la quantité de complexe formé entre l'ARN polymérase et la susdite protéine en présence d'un inhibiteur de référence,
et l'autre correspondant :

- soit à l'absence de complexe formé entre l'ARN polymérase et la protéine intervenant au cours de la transcription, résultant de la mise en présence de la protéine intervenant au cours de la transcription fixée sur un support, de l'ARN polymérase et d'un excès de protéine intervenant au cours de la transcription, l'excès de protéine intervenant au cours de la transcription étant de préférence préalablement incubé avec l'ARN polymérase, l'absence du susdit complexe correspondant à une inhibition totale de la complexation entre l'ARN polymérase et la susdite protéine,
- soit à l'absence de complexe formé entre l'ARN polymérase et la protéine intervenant au cours de la transcription, résultant de la présence seule d'ARN polymérase et de l'absence de protéine intervenant au cours de la transcription.

**6.** Procédé de détection selon l'une des revendications 1 à 5, dans lequel, au cours de l'étape de détection de la

variation significative de la quantité de complexe formé entre l'ARN polymérase et la protéine intervenant au cours de la transcription, on utilise un anticorps anti-ARN polymérase.

**7.** Procédé de détection selon l'une des revendications 1 à 6, dans lequel la protéine intervenant au cours de la transcription a un poids moléculaire supérieur à 15 kDa ou est une protéine de fusion entre une protéine de poids moléculaire inférieur à 15 kDa et une autre protéine telle que la GST (glutathione S transférase).

**8.** Procédé de détection selon l'une des revendications 1 à 7, dans lequel les concentrations de l'ARN polymérase sont comprises de 1 fmole à 100 pmole/essai, celles de la protéine intervenant au cours de la transcription de 10 fmole à 500 pmole/essai et celles du composé soumis au procédé de détection de 1 pM à 1 $\mu$M.

**9.** Procédé de détection selon l'une des revendications 1 à 8, dans lequel l'ARN polymérase utilisée provient de cellules procaryotes.

**10.** Procédé de détection selon l'une des revendications 1 à 9, dans lequel la protéine intervenant au cours de la transcription intervient soit au cours de l'étape d'initiation de la transcription, soit au cours de l'étape d'élongation, soit au cours de l'étape de terminaison de la transcription.

**11.** Procédé de détection selon l'une des revendications 1 à 10, dans lequel la protéine intervenant au cours de la transcription est :

- soit la protéine $\sigma^{70}$,
- soit la protéine NusA,
- soit une de ces deux protéines sous forme de protéine de fusion.

**12.** Procédé de détection d'un composé inhibiteur de la complexation entre l'ARN polymérase et la protéine $\sigma^{70}$ selon l'une quelconque des revendications 1 à 11, comprenant les étapes suivantes :

- une étape d'adsorption de la protéine $\sigma^{70}$ sur un support solide durant une nuit à 4°C, ladite protéine étant notamment à une concentration de 1 $\mu$M dans du tampon PBS (NaCl 150 mM ; $K_2PO_4$ 2,5 mM ; $Na_2PO_4$ 8,5 mM - pH 7,2),
- une étape de lavage dudit support solide avec du PBS-T 0,1% (v/v) (NaCl 150 mM ; $K_2PO_4$ 2,5 mM ; $Na_2PO_4$ 8,5 mM - pH 7,2 ; Tween 20 0,1 % (v/v)) pour éliminer tout ce qui ne s'est pas fixé sur la plaque,
- une étape de saturation dudit support solide avec 200 $\mu$l de solution de saturation (PBS-T 0,1% (v/v) ; BSA 1% (p/v)) pendant une heure à température ambiante, afin d'éviter toute réaction non spécifique,
- une étape d'élimination de la solution de saturation,
- une étape d'addition au support solide de l'ARN polymérase et d'un composé soumis au procédé de détection à une concentration de 20 $\mu$g/ml et une étape d'incubation dudit support solide pendant une heure à température ambiante,
- une étape de lavage du support solide trois fois avec du PBS-T 0,1% (v/v),
- une étape de détection de la variation significative de la quantité de complexe formé entre l'ARN polymérase et la protéine $\sigma^{70}$ par rapport à une valeur de contrôle correspondant à la quantité de complexe formé entre l'ARN polymérase et la protéine $\sigma^{70}$ en l'absence de tout modulateur, cette étape comprenant l'utilisation d'un anticorps monoclonal dirigé contre la sous-unité $\beta$ de l'ARN polymérase et couplé à la peroxydase dans la solution de saturation, l'incubation dudit anticorps couplé à la peroxydase pendant 30 minutes à température ambiante, l'addition du substrat de la peroxydase et la mesure de l'absorbance à 490 nm après arrêt de la réaction entre la peroxydase et son substrat, et
- une étape de déduction, lorsqu'il y a une variation significative telle que définie ci-dessus, de la formation d'une liaison entre d'une part le susdit composé et d'autre part l'ARN polymérase et/ou la protéine $\sigma^{70}$, ce qui se traduit par une modulation de la complexation entre l'ARN polymérase et la protéine $\sigma^{70}$.

**13.** Procédé de détection d'un composé inhibiteur de la complexation entre l'ARN polymérase et la protéine NusA selon l'une quelconque des revendications 1 à 11, comprenant les étapes suivantes :

- une étape d'adsorption de la protéine NusA sur un support solide durant une nuit à 4°C, ladite protéine étant notamment à une concentration de 1 $\mu$M dans du tampon PBS (NaCl 150 mM ; $K_2PO_4$ 2,5 mM ; $Na_2PO_4$ 8,5 mM - pH 7,2),
- une étape de lavage dudit support solide avec du PBS-T 0,1% (v/v) (NaCl 150 mM ; $K_2PO_4$ 2,5 mM ; $Na_2PO_4$

8,5 mM - pH 7,2 ; Tween 20 0,1% (v/v)) pour éliminer tout ce qui ne s'est pas fixé sur la plaque,
- une étape de saturation dudit support solide avec 200 μl de solution de saturation (PBS-T 0,1% (v/v) ; BSA 1% (p/v)) pendant une heure à température ambiante, afin d'éviter toute réaction non spécifique,
- une étape d'élimination de la solution de saturation,
- une étape d'addition au support solide de l'ARN polymérase et d'un composé soumis au procédé de détection à une concentration de 20 μg/ml et une étape d'incubation dudit support solide pendant une heure à température ambiante dans un mélange NaCl 350 mM - $K_2PO_4$ 2,5 mM - $Na_2PO_4$ 8,5 mM ; pH 7,2,
- une étape de lavage du support solide trois fois avec du PBS-T 0,1% (v/v),
- une étape de détection de la variation significative de la quantité de complexe formé entre l'ARN polymérase et la protéine NusA par rapport à une valeur de contrôle correspondant à la quantité de complexe formé entre l'ARN polymérase et la protéine NusA en l'absence de tout modulateur, cette étape comprenant l'utilisation d'un anticorps monoclonal dirigé contre la sous-unité β de l'ARN polymérase et couplé à la peroxydase dans la solution de saturation, l'incubation dudit anticorps couplé à la peroxydase pendant 30 minutes à température ambiante, l'addition du substrat de la peroxydase et la mesure de l'absorbance à 490 nm après arrêt de la réaction entre la peroxydase et son substrat, et
- une étape de déduction, lorsqu'il y a une variation significative telle que définie ci-dessus, de la formation d'une liaison entre d'une part le susdit composé et d'autre part l'ARN polymérase et/ou la protéine NusA, ce qui se traduit par une inhibition de la complexation entre l'ARN polymérase et la protéine NusA.

## Claims

**1.** Process for the detection of a compound modulating the complexation between RNA polymerase and a protein intervening during the transcription, in which:

- the protein intervening during the transcription is deposited on a solid support, to adsorb said protein on the solid support,
- the RNA polymerase and the compound subjected to the detection process are added to said solid support,
- said solid support comprising the RNA polymerase, the protein intervening during the transcription and the compound subjected to the detection process are incubated, and a complex between the RNA polymerase and said protein is formed and a bond, on the one hand between said compound and on the other hand the RNA polymerase, and/or the protein intervening during the transcription is formed,
- by means of a complexation test other than a chromatography or an immunoprecipitation, any significant variation in the quantity of complex formed between the RNA polymerase and said protein is detected, with respect to a control value corresponding to the quantity of complex formed between the RNA polymerase and said protein in the absence of any modulator, said significant variation corresponding to a variation of about more than 20% of the quantity of complex formed, and
- when there is a significant variation as defined above, it is deduced from this that a bond has been formed between on the one hand said compound and on the other hand the RNA polymerase, and/or the protein intervening during the transcription, which is translated by a modulation of the complexation between the RNA polymerase and the protein intervening during the transcription.

**2.** Detection process according to claim 1, in which the modulating compound is a compound activating the complexation between RNA polymerase and a protein intervening during the transcription, and in which:

- the protein intervening during the transcription is deposited on a solid support, to adsorb said protein on the solid support,
- the RNA polymerase and the compound subjected to the detection process are added to said solid support,
- said solid support comprising the RNA polymerase, the protein intervening during the transcription and the compound subjected to the detection process are incubated, and a complex between the RNA polymerase and said protein is formed and a bond, on the one hand between said compound and on the other hand the RNA polymerase, and/or the protein intervening during the transcription is formed,
- by means of a complexation test other than a chromatography or an immunoprecipitation, any significant variation in the quantity of complex formed between the RNA polymerase and said protein is detected, with respect to a control value corresponding to the quantity of complex formed between the RNA polymerase and said protein in the absence of any activator, said significant variation corresponding to an increase of at least 120% of the quantity of complex formed, and
- when there is a significant variation as defined above, it is deduced from this that a bond has been formed

between on the one hand said compound and on the other hand the RNA polymerase, and/or the protein intervening during the transcription, which is translated by an activation of the complexation between the RNA polymerase and the protein intervening during the transcription.

**3.** Detection process according to claim 1, in which the modulating compound is a compound inhibiting the complexation between RNA polymerase and a protein intervening during the transcription, and in which:

- the protein intervening during the transcription is deposited on a solid support, to adsorb said protein on the solid support,
- the RNA polymerase and the compound subjected to the detection process are added to said solid support,
- said solid support comprising the RNA polymerase, the protein intervening during the transcription and the compound subjected to the detection process are incubated, and a complex between the RNA polymerase and said protein is formed and a bond, on the one hand between said compound and on the other hand the RNA polymerase, and/or the protein intervening during the transcription is formed,
- by means of a complexation test other than a chromatography or an immunoprecipitation, any significant variation in the quantity of complex formed between the RNA polymerase and said protein is detected, with respect to a control value corresponding to the quantity of complex formed between the RNA polymerase and said protein in the absence of any inhibitor, said significant variation corresponding to a decrease of about more than 20% of the quantity of complex formed, and
- when there is a significant variation as defined above, it is deduced from this that a bond has been formed between on the one hand said compound and on the other hand the RNA polymerase, and/or the protein intervening during the transcription, which is translated by an inhibition of the complexation between the RNA polymerase and the protein intervening during the transcription.

**4.** Detection process according to claim 3, comprising the use of two control values, one corresponding to the incubation of the RNA polymerase alone with the protein intervening during the transcription in the absence of any inhibitor (which corresponds to an absence of inhibition) and the other corresponding to an incubation of the RNA polymerase with the protein intervening during the transcription and with a reference inhibitor (which corresponds to a reference inhibition).

**5.** Detection process according to claim 3, comprising the use of a first control value and/or of a second control value and/or of a third control value,

one corresponding to the quantity of complex formed between the RNA polymerase and said protein in the absence of any inhibitor,
the other corresponding to the quantity of complex formed between the RNA polymerase and said protein in the presence of a reference inhibitor,
and the other corresponding:

- either to the absence of complex formed between the RNA polymerase and the protein intervening during the transcription, resulting from the introduction of the protein intervening during the transcription fixed on a support, of the RNA polymerase and of an excess of protein intervening during the transcription, the excess of protein intervening during the transcription preferably being previously incubated with the RNA polymerase, the absence of said complex corresponding to a total inhibition of the complexation between the RNA polymerase and said protein,
- or to the absence of complex formed between the RNA polymerase and the protein intervening during the transcription, resulting from the presence of RNA polymerase alone and from the absence of protein intervening during the transcription.

**6.** Detection process according to one of claims 1 to 5, in which, during the stage of detection of any significant variation in the quantity of complex formed between the RNA polymerase and the protein intervening during the transcription, an anti-RNA polymerase antibody is used.

**7.** Detection process according to one of claims 1 to 6, in which the protein intervening during the transcription has a molecular weight greater than 15 kDa or is a fusion protein between a protein with a molecular weight of less than 15 kDa and another protein such as GST (glutathione S transferase).

**8.** Detection process according to one of claims 1 to 7, in which the RNA polymerase concentrations are comprised

from 1 fmole to 100 μmole/test, those of the protein intervening during the transcription from 10 fmole to approximately 500 pmole/test and those of the compound subjected to the detection process from 1 μM to 1 μM.

**9.** Detection process according to one of claims 1 to 8, in which the RNA polymerase used originates from prokaryotic cells.

**10.** Detection process according to one of claims 1 to 9, in which the protein intervening during the transcription intervenes either during the transcription initiation stage, or during the elongation stage, or during the transcription termination stage.

**11.** Detection process according to one of claims 1 to 10, in which the protein intervening during the transcription is:

- either the protein $\sigma^{70}$,
- either the protein NusA,
- or one of these two proteins in the form of a fusion protein,

**12.** Process for the detection of a compound inhibiting the complexation between RNA polymerase and the protein $\sigma^{70}$ according to one of claims 1 to 11, comprising the following steps:

- a step of adsorption of the protein $\sigma^{70}$on a microassay plate during one night at 4°C, said protein being in particular at a concentration of 1 μM in PBS buffer (150 mM NaCl; 2.5 mM $K_2PO_4$; 8.5 mM $Na_2PO_4$ - pH 7.2),
- a step of washing said solid support with 0.1% PBS-T (v/v) (150 mM NaCl; 2.5 mM $K_2PO_4$; 8.5 mM $Na_2PO_4$ - pH 7.2; Tween 20 0.1% (v/v)) in order to eliminate anything not fixed on the plate.
- a step of saturating the wells of said microassay plate with 200 μl of saturation solution (PBS-T 0.1% (v/v); 1% BSA (w/v)) for one hour at ambient temperature, in order to avoid any non-specific reaction,
- a step of eliminating the saturation solution,
- a step of adding to the microassay plate RNA polymerase and a compound subjected to the detection process at a concentration of 20 μg/ml and a step of incubating said microassay plate for one hour at ambient temperature,
- a step of washing said microassay plate three times with 0.1% PBS-T (v/v),
- a step of detecting the significant variation in the quantity of complex formed between the RNA polymerase and protein $\sigma^{70}$, with respect to a control value corresponding to the quantity of complex formed between the RNA polymerase and protein $\sigma^{70}$ in the absence of any modulator, this step comprising the use of a monoclonal antibody recognizing the β sub-unit of the RNA polymerase and coupled to the peroxidase in the saturation solution, incubating said antibody coupled to the peroxidase during 30 minutes at ambient temperature, adding the peroxidase substrate and measuring the absorbance at 490 nm after stopping the reaction between peroxidase and its substrate, and
- a step of deducing, when there is a significant variation as defined above, that a bond has been formed between on the one hand said compound and on the other hand the RNA polymerase, and/or the protein $\sigma^{70}$, which is translated by a modulation of the complexation between the RNA polymerase and the protein $\sigma^{70}$.

**13.** Process for the detection of a compound inhibiting the complexation between RNA polymerase and the protein NusA according one of claims 1 to 11, comprising the following steps:

- a step of adsorption of the protein NusA on a microassay plate during one night at 4°C, said protein being in particular at a concentration of 1 μM in PBS buffer (150 mM NaCl; 2.5 mM $K_2PO_4$; 8.5 mM $Na_2PO_4$ - pH 7.2),
- a step of washing said solid support with 0.1% PBS-T (v/v) (150 mM NaCl; 2.5 mM $K_2PO_4$; 8.5 mM $Na_2PO_4$ - pH 7.2; Tween 20 0.1% (v/v)) in order to eliminate anything not fixed on the plate.
- a step of saturating the wells of said microassay plate with 200 μl of saturation solution (PBS-T 0.1% (v/v); 1% BSA (w/v)) for one hour at ambient temperature, in order to avoid any non-specific reaction,
- a step of eliminating the saturation solution,
- a step of adding to the microassay plate RNA polymerase and a compound subjected to the detection process at a concentration of 20 μg/ml and a step of incubating said microassay plate for one hour at ambient temperature,
- a step of washing said microassay plate three times with 0.1% PBS-T (v/v),
- a step of detecting the significant variation in the quantity of complex formed between the RNA polymerase and protein NusA, with respect to a control value corresponding to the quantity of complex formed between the RNA polymerase and protein NusA in the absence of any modulator, this step comprising the use of a monoclonal antibody recognizing the β sub-unit of the RNA polymerase and coupled to the peroxidase in the saturation solution, incubating said antibody coupled to the peroxidase during 30 minutes at ambient temperature, adding

the peroxidase substrate and measuring the absorbance at 490 nm after stopping the reaction between peroxidase and its substrate, and
- a step of deducing, when there is a significant variation as defined above, that a bond has been formed between on the one hand said compound and on the other hand the RNA polymerase, and/or the protein NusA, which is translated by a modulation of the complexation between the RNA polymerase and the protein NusA.

**Patentansprüche**

1. Verfahren für das Auffinden von einer Verbindung, die die Komplexierung zwischen der RNA Polymerase und ein Protein, das während der Transkription eingreift, moduliert, worin :

   - Das Protein, das während der Transkription eingreift, wird auf einen festen Untergrund deponiert, und zwar um das Protein auf diesem festen Untergrund zu adsorbieren,
   - Die RNA Polymerase wird dem festen Untergrund zugefügt, und die Verbindung wird dem Erkennungsverfahren unterworfen,
   - Der Untergrund wird mit der RNA Polymerase und dem Protein, das während der Transkription eingreift, sowie der dem Erkennungsverfahren unterworfenen Verbindung, inkubiert, und ein Komplex wird gebildet, und zwar zwischen der RNA Polymerase und das obengenannte Protein, sowie eine Bindung zwischen der obengenannten Verbindung einerseits und der RNA Polymerase und/oder dem Protein, das während der Transkription eingreift, andererseits,
   - Die bedeutsame Schwankung in der Menge des Komplexes zwischen der RNA Polymerase und dem obengenannten Protein in Vergleich zu einem Kontrollwert, das mit der Komplexmenge, die zwischen der RNA Polymerase und dem obengenannten Protein in Abwesenheit von jedem Modulator gebildet wird, übereinstimmt, wird mit einem Komplexierungstest, der anders ist, als eine Chromatographie oder eine Immunpräzipitation detektiert, worin die obengenannte bedeutsame Schwankung eine Variation von ca. mehr als 20 % der gebildeten Komplexmenge bedeutet, und
   - Es wird davon deduziert, wenn eine wie oben definierte bedeutsame Schwankung stattgefunden hat, daß eine Bindung zwischen dem obengenannten Verbindung einerseits und der RNA Polymerase und/oder dem Protein, das während der Transkription eingreift andererseits, gebildet wurde, was eine Modulation der Komplexierung zwischen der RNA Polymerase und dem Protein, das während der Transkription eingreift, zur Folge hat.

2. Ein Detektionsverfahren nach Anspruch 1, worin die modulatorische Verbindung eine aktivatorische Verbindung für die Komplexierung zwischen der RNA Polymerase und einem Protein, daß während der Transkription eingreift, ist, und worin :

   - Das Protein, das während der Transkription eingreift, wird auf einen festen Untergrund deponiert, um dieses Protein auf dem festen Untergrund zu adsorbieren,
   - Die RNA Polymerase und die Verbindung, die dem Detektionsverfahren unterworfen wird, werden dem festen Untergrund hinzugefügt,
   - Der obergenannte feste Untergrund wird mit der RNA Polymerase, dem Protein, das während der Transkription eingreift, und der Verbindung, die dem Detektionsverfahren unterworfen wurde, inkubiert, und ein Komplex zwischen der RNA Polymerase und dem obengenannten Protein, sowie eine Bindung zwischen der obengenannten Verbindung einerseits, und der RNA Polymerase und/oder dem Protein, das während der Transkription eingreift, andererseits, gebildet wird,
   - Die bedeutsame Schwankung in der Menge des Komplexes zwischen der RNA Polymerase und dem obengenannten Protein im Vergleich zu einem Kontrollwert, das mit der Komplexmenge, die zwischen der RNA Polymerase und dem obengenannten Protein in Abwesenheit von jedem Aktivator gebildet wird, übereinstimmt, wird mit einem Komplexierungstest, der anders ist, als eine Chromatographie oder eine Immunpräzipitation detektiert, worin die obengenannte bedeutsame Schwankung eine Erhöhung von mindestens 120 % der gebildeten Komplexmenge bedeutet, und
   - Es wird davon deduziert, wenn eine wie oben definierte bedeutsame Schwankung stattgefunden hat, daß eine Bindung zwischen der obengenannten Verbindung einerseits und der RNA Polymerase und/oder dem Protein, das während der Transkription eingreift andererseits, gebildet wurde, was eine Aktivierung der Komplexierung zwischen der RNA Polymerase und dem Protein, das während der Transkription eingreift, zur Folge hat.

3. Ein Verfahren nach Anspruch 1, worin die modulatorische Verbindung eine Hemmungsverbindung für die Komplexierung zwischen der RNA Polymerase und einem Protein, das während der Transkription eingreift, ist, und worin :

- Das Protein, das während der Transkription eingreift, wird auf einen festen Untergrund deponiert, um dieses Protein auf dem festen Untergrund zu adsorbieren,

- Die RNA Polymerase und die Verbindung, die dem Detektionsvervahren unterworfen wird, werden dem festen Untergrund hinzugefügt,

- Der obengenannte feste Untergrund wird mit der RNA Polymerase, dem Protein, das während der Transkription eingreift und der dem Erkennungsverfahren unterworfenen Verbindung inkubiert, und einen Komplex zwischen der RNA Polymerase und dem obengenannten Protein, sowie eine Bindung zwischen der obengenannten Verbindung einerseits, und der RNA Polymerase und/oder dem Protein, das während der Transkription eingreift, andererseits, gebildet wird,

- Die bedeutsame Schwankung in der Menge des Komplexes zwischen der RNA Polymerase und dem obengenannten Protein im Vergleich zu einem Kontrollwert, das mit der Komplexmenge, die zwischen der RNA Polymerase und dem obengenannten Protein in Abwesenheit von jedem Hemmer gebildet wird, übereinstimmt, wird mit einem Komplexierungstest, der anders ist, als eine Chromatographie oder eine Immunpräzipitation detektiert, worin die obengenannte bedeutsame Schwankung eine Erniedrigung von ca. mehr als 20 % der gebildeten Komplexmenge bedeutet, und

- Es wird davon deduziert, wenn eine wie oben definierte bedeutsame Schwankung stattgefunden hat, daß eine Bindung zwischen der obengenannten Verbindung einerseits und der RNA Polymerase und/oder dem Protein, das während der Transkription eingreift andererseits, gebildet wurde, was eine Hemmung der Komplexierung zwischen der RNA Polymerase und dem Protein, das während der Transkription eingreift, zur Folge hat.

**4.** Ein Verfahren für die Detektion nach Anspruch 3, worin zwei Kontrollwerte benutzt werden, und zwar einen Wert, der der Inkubation von RNA Polymerase allein mit dem Protein entspricht, das während der Transkription in Abwesenheit von jedem Hemmer eingreift (d.h. in Abwesenheit von jeder Hemmung), und den anderen Wert, der einer Inkubation von der RNA Polymerase mit dem Protein entspricht, das während der Transkription eingreift, und mit einem Referenzhemmer (d.h. mit einer Referenzhemmung).

**5.** Ein Verfahren für die Detektion nach Anspruch 3, worin einen ersten Kontrollwert und/oder einen zweiten Kontrollwert und/oder einen dritten Kontrollwert benutzt werden,
worin der eine der Menge des zwischen der RNA Polymerase und dem obengenannten Protein in Abwesenheit von jeden Hemmer gebildeten Komplexes entspricht,
der zweite der Menge des zwischen der RNA Polymerase und dem obengenannten Protein in Anwesenheit von einem Referenzhemmer gebildeten Komplexes entspricht,
und der dritte :

- entweder der Abwesenheit von einem Komplex zwischen der RNA Polymerase und dem Protein, das während der Transkription eingreift, worin diese Abwesenheit das Ergebnis der Konfrontation des Proteins, das während der Transkription eingreift, und das auf einen Untergrund gebindet wird, der RNA Polymerase und eines Übermaßes vom Protein, das während der Transkription eingreift, ist, und worin das Übermaß vom Protein, das während der Transkription eingreift, zunächst mit der RNA Polymerase inkubiert wird, und auch worin die Abwesenheit des obengenannten Komplexes einer totalen Hemmung der Komplexierung zwischen der RNA Polymerase und dem obengenannten Protein entspricht,

- oder der Abwesenheit von einem Komplex zwischen der RNA Polymerase und dem Protein, das während der Transkription eingreift, worin diese Abwesenheit der einzigen Anwesenheit von RNA Polymerase und der Abwesenheit des Protein, das während der Transkription eingreift, entspricht.

**6.** Ein Verfahren für die Detektion nach irgendwelcher Anspruch 1 - 5, worin während des Detektionsschritts der bedeutsamen Schwankung der Komplexmenge, die zwischen der RNA Polymerase und dem Protein, das während der Transkription eingreift, gebildet wird, ein Anti-RNA Polymerase Antikörper verwendet wird.

**7.** Ein Verfahren für die Detektion nach irgendwelcher Anspruch 1 - 6, worin das Protein, das während der Transkription eingreift, ein Molekulargewicht über 15 kDa hat, oder ein Fusionsprotein zwischen einem Protein mit einem Molekulargewicht unter 15 kDa und einem anderen Protein wie GST (Glutathion S Transferase) ist.

**8.** Ein Verfahren für die Detektion nach irgendwelcher Anspruch 1 - 7, worin die Konzentrationen von RNA Polymerase zwischen 1 fMol und 100 pMol/Test, die dem Protein, das während der Transkription eingreift, zwischen 10 fMol und 500 pMol/Test und die der Verbindung, die dem Detektionsverfahren unterworfen wird, zwischen 1 pM und 1 μM mit eingeschlossen sind.

**9.** Ein Verfahren für die Detektion nach irgendwelcher Anspruch 1 - 8, worin die RNA Polymerase, die verwendet wird, aus prokaryontischen Zellen kommt.

**10.** Ein Verfahren für die Detektion nach irgendwelcher Anspruch 1 - 9, worin das Protein, das während der Transkription eingreift, entweder während des Initiationsschrittes der Transkription oder während des Verlängerungsschrittes, oder während des Transkriptionsbeendigungschrittes wirkt.

**11.** Ein Verfahren für die Detektion nach irgendwelcher Anspruch 1 - 10, worin das Protein, das während der Transkription eingreift :

- entweder das $\sigma^{70}$ Protein,
- oder das NusA Protein,
- oder irgendwelches von diesen zwei Proteinen in der Gestalt eines Fusionsproteins ist.

**12.** Ein Verfahren für die Detektion einer Hemmerverbindung für die Komplexierung zwischen der RNA Polymerase und dem $\sigma^{70}$ Protein nach irgendwelcher Anspruch 1 - 11, das die folgenden Schritte mit einschließt:

- Einen Adsorptionsschritt für das Protein $\sigma^{70}$ auf einen festen Untergrund während einer Nacht bei 4 °C, worin das obengenannte Protein bei einer Konzentration von 1 $\mu$M in PBS Puffer (NaCl 150 mM ; $K_2PO_4$ 2,5 mM ; $Na_2PO_4$ 8,5 mM - pH 7,2) liegt,
- Einen Waschschritt für diesen festen Untergrund mit PBS-T 0,1 % (v/v) (NaCl 150 mM ; $K_2PO_4$ 2,5 mM ; $Na_2PO_4$ 8,5 mM ; - pH 7,2 ; Tween 20 0,1 % (v/v) ) um alles, was sich nicht auf die Plaque abgelagert hat, zu beseitigen,
- Einen Sättigunsschritt von diesem festen Untergrund mit 200 $\mu$l Sättigunslösung (PBS-T 0,1 % (v/v) ; BSA 1 % (p/v) ) während einer Stunde bei Raumtemperatur, um irgendwelche unspezifische Reaktion zu vermeiden,
- Einen Beseitigungsschritt für die Sättigunslösung,
- Einen Zugabesschritt, zum festen Untergrund, von der RNA Polymerase und von einer Verbindung, die dem Detektionsverfahren bei einer Konzentration von 20 $\mu$g/ml untergeworfen wird, und einen Inkubationsschritt des obengenannten festen Untergrund während einer Stunde bei Raumtemperatur,
- Einen Waschsschritt des festen Untergrunds, und zwar dreimal mit PBS-T 0,1 % (v/v), Einen Detektionsschritt für die bedeutsame Schwankung der gebildeten Komplexmenge zwischen RNA Polymerase und Protein $\sigma^{70}$ im Vergleich mit einem Kontrollwert, das mit der Komplexmenge, die zwischen der RNA Polymerase und dem Protein $\sigma^{70}$ in Abwesenheit von jedem Modulator gebildet wird, übereinstimmt, worin dieser Schritt die Verwendung eines monoklonalen Antikörper gegen die $\beta$ Subeinheit der RNA Polymerase, der auch mit der Peroxydase in der Sättigunslösung gekoppelt wird, die Inkubation vom obengenannten Antikörper, der mit der Peroxydase gekoppelt wird, und zwar während 30 Minuten bei Raumtemperatur, die Zugabe des Peroxydasesubstrats und die Messung der Absorptionsfähigkeit bei 490 nm nach Ende der Reaktion zwischen der Peroxydase und ihrer Substrat mit einschließt, und
- Einen Deduktionsschritt, wenn eine wie oben definierte bedeutsame Schwankung besteht, der Bildung einer Bindung zwischen der obengenannten Verbindung einerseits, und der RNA Polymerase und/oder dem Protein $\sigma^{70}$ andererseits, was eine Modulation der Komplexierung zwischen der RNA Polymerase und dem Protein $\sigma^{70}$ zur Folge hat.

**13.** Ein Verfahren für die Detektion einer Hemmerverbindung für die Komplexierung zwischen der RNA Polymerase und dem Protein NusA nach irgendwelcher Anspruch 1 - 11, das die folgenden Schritte mit einschließt :

- Einen Adsorptionsschritt für das Protein NusA auf einen festen Untergrund während einer Nacht bei 4 °C, worin das obengenannte Protein besonders bei einer Konzentration von 1 mM in PBS Puffer (NaCl 150 mM ; $K_2PO_4$ 2,5 mM ; $Na_2PO_4$ 8,5 mM - pH 7,2) vorliegt,
- Einen Waschsschritt für den obengenannten Untergrund mit PBS-T 0,1 % (v/v) (NaCl 150 mM ; $K_2PO_4$ 2,5 mM ; $Na_2PO_4$ 8,5 mM - pH 7,2 ; Tween 20 0,1 % (v/v) ) um alles, was sich nicht auf die Plaque abgelagert hat, zu beseitigen,
- Einen Sättigungsschritt des obengenannten festen Untergrund mit 200 $\mu$l Sättigungslösung (PBS-T 0,1 % (v/v) ; BSA 1 % (Gew./v)) während einer Stunde bei Raumtemperatur, um jede unspezifische Reaktion zu vermeiden,
- Einen Beseitigunsschritt für die Sättigungslösung,
- Einen Zugabeschritt, zum festen Untergrund, von der RNA Polymerase und von einer Verbindung, die dem Detektionsverfahren bei einer Konzentration von 20 $\mu$g/l untergeworfen wird, und einen Inkubationsschritt des

obengenannten Untergrund während einer Stunde bei Raumtemperatur in einer Mischung von NaCl 350 mM - $K_2PO_4$ 2,5 mM -$Na_2PO_4$ 8,5 mM ; pH 7,2,

- Einen Waschsschritt des festen Untergrund, und zwar dreimal mit PBS-T 0,1 % (v/v),
- Einen Detektionsschritt für die bedeutsame Schwankung der Komplexmenge zwischen RNA Polymerase und Protein NusA im Vergleich mit einem Kontrollwert, das mit der Komplexmenge, die zwischen der RNA Polymerase und dem Protein NusA in Abwesenheit von jedem Modulator gebildet wird, übereinstimmt, worin dieser Schritt die Verwendung eines monoklonalen Antikörpers gegen die β Subeinheit der RNA Polymerase, der auch mit der Peroxydase in der Sättigungslösung gekoppelt wird, die Inkubation vom obengenannten Antikörper, der mit der Peroxydase gekoppelt wird, und zwar während 30 Minuten bei Raumtemperatur, die Zugabe des Peroxydasesubstrats und die Messung der Absorptionsfähigkeit bei 490 nm nach Ende der Reaktion zwischen der Peroxydase und ihrer Substrat mit einschließt, und
- Einen Deduktionsschritt, wenn eine wie oben definierte bedeutsame Schwankung besteht, der Bildung einer Bindung zwischen der obengenannten Verbindung einerseits, und der RNA Polymerase und/oder dem Protein NusA andererseits, was eine Hemmung der Komplexierung zwischen der RNA Polymerase und dem Protein NusA zur Folge hat.

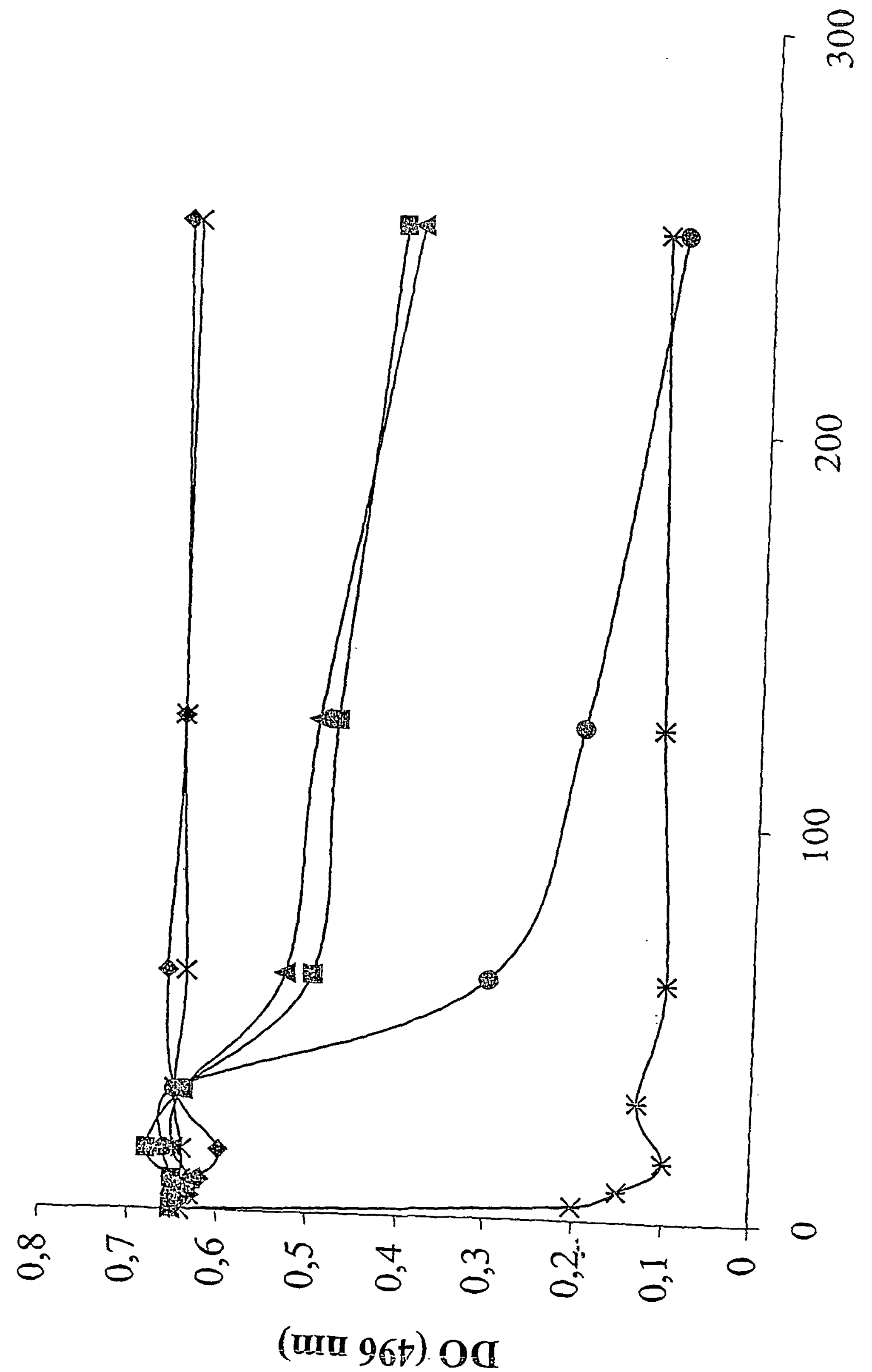
DO (496 nm)
0,8
0,7
0,6
0,5
0,4
0,3
0,2
0,1
0
0
100
200
300
concentration (µg/ml)

FIGURE 1

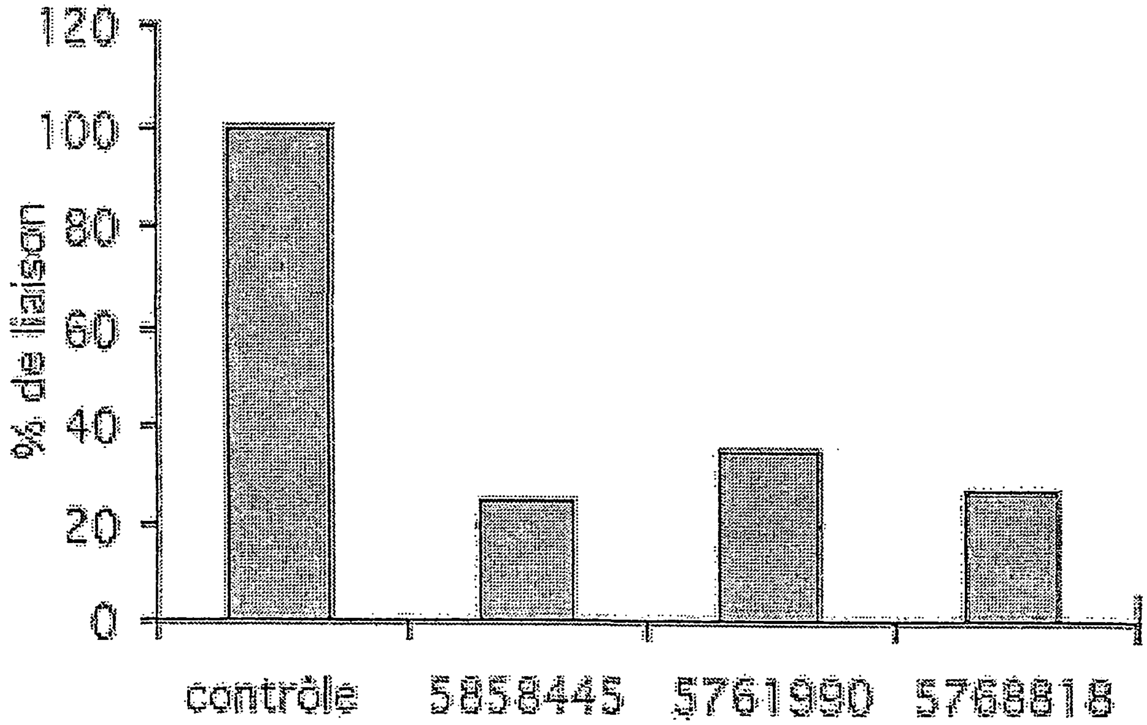
% de liaison
0
20
40
60
80
100
120
contrôle
5858445
5761990
5768818

FIGURE 2

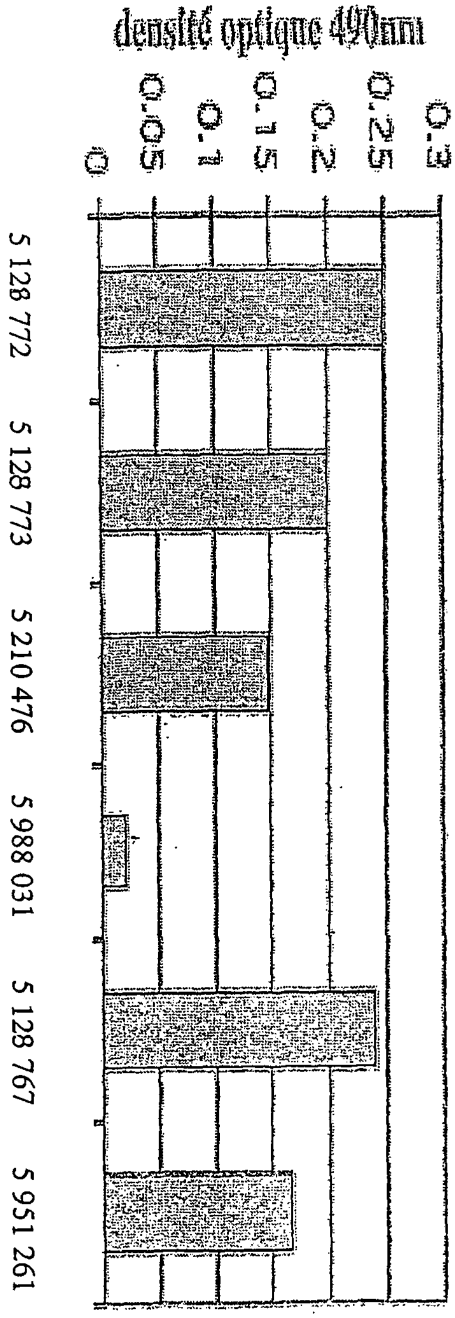
densité optique 490nm
0
0.05
0.1
0.15
0.2
0.25
0.3
5 128 772
5 128 773
5 210 476
5 988 031
5 128 767
5 951 261

FIGURE 3

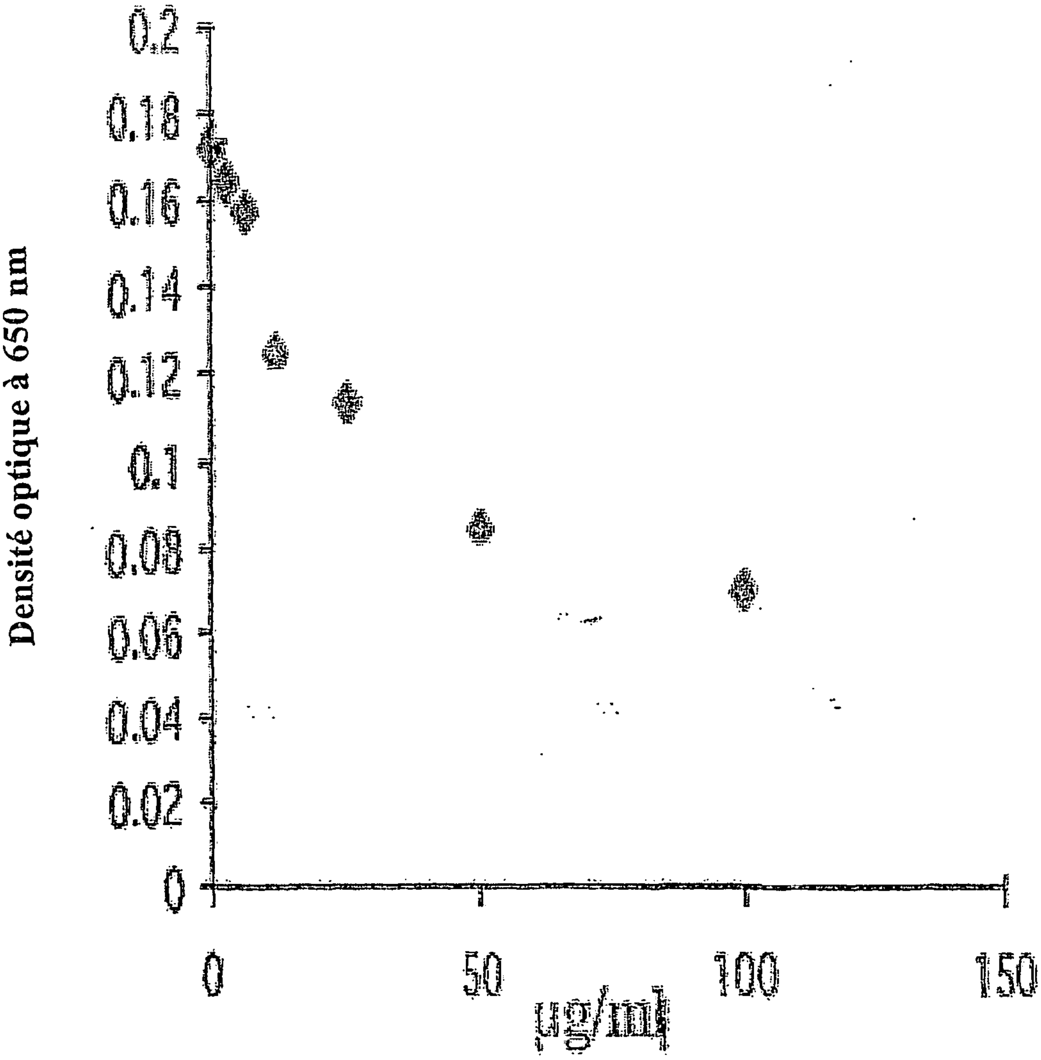
Densité optique à 650 nm
0.2
0.18
0.16
0.14
0.12
0.1
0.08
0.06
0.04
0.02
0
0
50
100
150
µg/ml

FIGURE 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9638478 A


### Littérature non-brevet citée dans la description


- **BURGESS et al.** *Biochemistry,* 21 Octobre 1975, vol. 14 (21), 4634-8
- **BURGESS R.** *Methods Enzymol,* 1996, vol. 273, 145-9
- Short Protocols in Molecular Biology. John Wiley & Son
- **BURGESS et al.** *Nature,* 1969, vol. 221, 43-44
- **BURGESS.** *Methods Enzymol,* 1996, vol. 273, 145-9
- **COURVALIN.** *Antimicrob Chemother,* 1996, vol. 37, 855-69
- **KOLESKY et al.** *J Mol Biol,* 1999, vol. 291, 267-81
- **LESLEY et al.** *Biochemistry,* 1989, vol. 28, 7728-7734
- **MUELLER, J.H. ; HINTON, J.** A protein-free medium for primary isolation of gonococcus and meningococcus. *Proc. Soc. Exp. Biol. Med.,* 1941, vol. 48, 330-333
- **REZNIKOFF et al.** *Annu. Rev. Genet.,* 1985, vol. 19, 355-387
- **WU et al.** *Analytical Biochemistry,* 1997, vol. 245, 226-230
- **YANG et al.** *J Biol Chem,* 1995, vol. 270, 23930-3